Europäisches Patentamt

European Patent Office

Office européen des brevets

(19)

(11) EP 0 944 567 B1

(12) **EUROPÄISCHE PATENTSCHRIFT**

(45) Veröffentlichungstag und Bekanntmachung des Hinweises auf die Patenterteilung:
**04.04.2001   Patentblatt 2001/14**

(21) Anmeldenummer: **97953777.6**

(22) Anmeldetag: **10.12.1997**

(51) Int Cl.⁷: **C07C 39/04**, C07C 49/08

(86) Internationale Anmeldenummer:
**PCT/EP97/06905**

(87) Internationale Veröffentlichungsnummer:
**WO 98/27039 (25.06.1998 Gazette 1998/25)**

(54) **VERBESSERTES VERFAHREN ZUR HERSTELLUNG VON PHENOL UND ACETON AUS CUMOL**

IMPROVED METHOD FOR PRODUCING PHENOL AND ACETONE FROM CUMOL

PROCEDE PERFECTIONNE POUR LA PREPARATION DE PHENOL ET D'ACETONE A PARTIR DE CUMENE

(84) Benannte Vertragsstaaten:
**BE DE ES FI FR GB IT NL PT SE**

(30) Priorität: **15.12.1996   RU 96123606**

(43) Veröffentlichungstag der Anmeldung:
**29.09.1999   Patentblatt 1999/39**

(73) Patentinhaber:
• **ILLA INTERNATIONAL LTD**
  **St. Petersburg, 193148 (RU)**
• **Phenolchemie GmbH & Co. KG**
  **45966 Gladbeck (DE)**

(72) Erfinder:
• **ZAKOSHANSKY, Vladimir Michailovitch**
  **St.Petersburg, 194356 (RU)**
• **VASILIEVA, Irina Ivanova**
  **St.Petersburg, 199397 (RU)**
• **GRIAZNOV, Andrei Konstantinovitch**
  **St.Petersburg, 191194 (RU)**

• **YOURIEV, Youry Nikolaevitch**
  **North Miami Beach, FL 33160 (US)**
• **VAN BARNEFELD, Heinrich**
  **D-46244 Bottrop (DE)**
• **GERLICH, Otto**
  **D-45966 Gladbeck (DE)**
• **KLEINE-BOYMANN, Michael**
  **D-46244 Bottrop (DE)**
• **KLEINLOH, Werner**
  **D-45721 Haltern (DE)**
• **MICHALIK, Christian**
  **D-45355 Essen (DE)**

(74) Vertreter: **Grussdorf, Jürgen, Dr. et al**
**Patentanwälte Zellentin & Partner**
**Rubensstrasse 30**
**67061 Ludwigshafen (DE)**

(56) Entgegenhaltungen:
**DE-A- 1 915 480          US-A- 5 530 166**

**Beschreibung**

Die Erfindung betrifft ein verbessertes Verfahren zur Herstellung von Phenol und Aceton unter Oxidation von Cumol mit nachfolgender Spaltung von Cumolhydroperoxid in einem Reaktor mit einem sauren Katalysator und destillativer Trennung der Reaktionsprodukte.

Alle existierenden Verfahren zur Herstellung von Phenol und Aceton aus Cumol bestehen aus den folgenden wichtigsten Stufen:

1. Oxidation von Cumol zum Cumolhydroperoxid (CHP).
2. Destillation der Oxidationsprodukte und Herstellung des technischen CHP.
3. Saure Spaltung des CHP und des darin enthaltenen Dimethylphenylcarbinol (DMPC) zu den Endprodukten Phenol, Aceton und α-Methylstyrol (AMS).
4. Neutralisation des sauren Katalysators und Abtrennung der Salze von den Reaktionsprodukten.
5. Trennung und Reinigung der Reaktionsprodukte.

In der Regel wurden die Forschungen bezüglich des Prozesses der Herstellung von Phenol mit dem Ziel der Verbesserung von nur einer Stufe des Prozesses durchgeführt. Dabei hat man nicht versucht, die Selektivität, Energiekennzahl und Sicherheit des Prozesses möglichst gleichzeitig zu verbessern. Die wichtigste Kennzahl ist der Dampfverbrauch.

Da die Hauptmasse unerwünschter Nebenprodukte in der Stufe der sauren Spaltung des CHP gebildet wird, beachten die meisten Forscher in erster Linie gerade diese Stufe. Im Laufe der Spaltung des technischen CHP, das reaktionsfähiges DMPC enthält, treten chemische Reaktionen auf, die zum Verlust an Endprodukten und zur Bildung von schwer verwertbaren Abfallprodukten, dem sogenannten phenolischen Teer, führen.

Das wichtigste Kriterium der Selektivität des Prozesses ist die Ausbeute an wertvollem Nebenprodukt, insbesondere α—Methylstyrol (AMS). Bekanntlich läßt sich AMS zu Cumol hydrieren oder als Produkt direkt isolieren.

Zur Steigerung der Ausbeute an AMS gibt es verschiedene Möglichkeiten:

a) Auswahl der Zusammensetzung des Reaktionsmediums (chemische Variante).

b) Auswahl des Reaktortyps und der Art der Wärmeableitung (technologische Variante).

c) Vereinigung der chemischen und technologischen Varianten.

Unsere Untersuchungen haben gezeigt, daß sich die katalytischen Eigenschaften der Schwefelsäure, die als Katalysator benutzt wird, im Phenol-Aceton-Reaktionsmedium durch die Bildung starker Wasserstoffbrückenbindungen zwischen den Phenolmolekülen sowie zwischen den Phenol- und Acetonmolekülen durch die Veränderung des Verhältnisses Phenol-Aceton und durch Zusatz eines inerten Lösungsmittels wie Cumol um ein Vielfaches ändern. Die Veränderung der Aktivität des Katalysators wird auch durch Wasser nicht weniger stark beeinflußt.

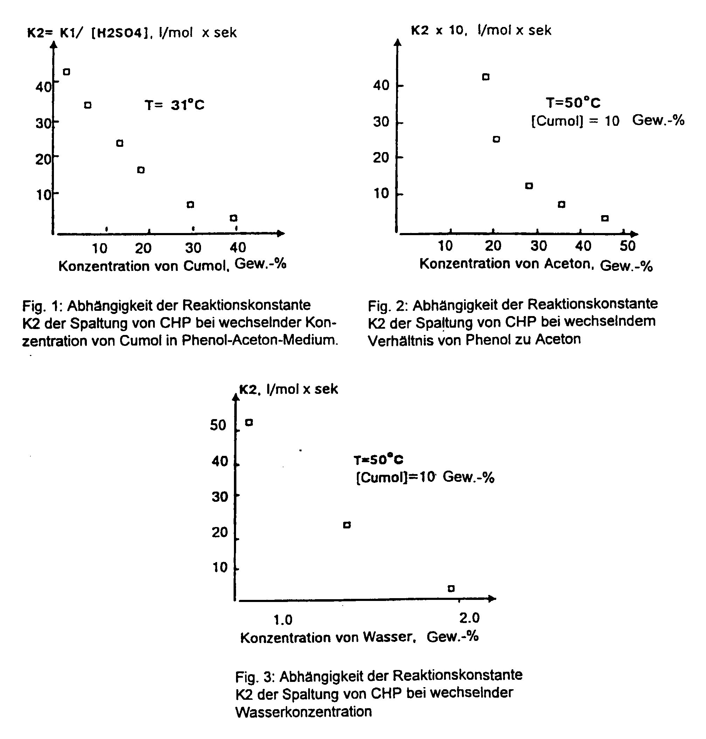

Kombinationen wechselnder Verhältnisse Phenol : Aceton : Wasser: Cumol: Schwefelsäure führen zu qualitativen Veränderungen der katalytischen Eigenschaften dieses Vielkomponenten-Reaktionsmediums, wie die Analyse unserer experimentellen Daten zeigt, die in Fig. 1, 2, 3 dargestellt ist.

Diese bedeutenden Veränderungen der Reaktionskonstanten der CHP -Spaltung hängen damit zusammen, daß große Änderungen der katalytischen Aktivität der Schwefelsäure in der Vielkomponenten-Lösung stattfinden. Diese Erscheinungen beweisen den Übergang von einem zu einem anderen Typ Katalysator. Eigentlich handelt es sich um den Übergang von einem superstarken (magic acid) zu einem schwachen Katalysator. Hieraus werden die großen Unterschiede in der Selektivität in den verschiedenen Patenten und Technologien verständlich, bei denen die Zusammensetzung des Reaktionsmediums verändert wird. So beträgt die Ausbeute an AMS ungefähr 40 - 45 % d. Th. bei einem Reaktionsmedium aus einem äquimolaren Gemisch von Phenol und Aceton (RU 1,361,937).

Im Prozeß gemäß der US 2,663,735, wo ein großer Überschuß an Aceton benutzt wird und das Molverhältnis Aceton : Phenol = 5 : 1 ist, übersteigt die Ausbeute an AMS 55 % d. Th. nicht.

Im Prozeß gemäß der RO 63,168 benutzt man als Reaktionsmedium auch ein äquimolares Gemisch von Phenol und Aceton, das bis zu 20 Gew.-% Cumol enthält. In diesem Fall beträgt die Ausbeute an AMS etwa 60 % d. Th..

Im Prozeß gemäß der RO 1,563,181 enthält das Reaktionsmedium einen Überschuß an Aceton von 20 % und nur 1 bis 2 % Cumol. In diesem Fall beträgt die Ausbeute an AMS schon etwa 70 % d. Th.

In den Prozessen gemäß der Patente US 5,254,751 und US 5,530,166 erreicht die Ausbeute an AMS um 80 % d. Th. In diesen Prozessen verwendet man ein Reaktionsmedium, das bis zu 20 Gew.-% Cumol und ein Molverhältnis von Aceton : Phenol = 1,5 : 1 enthält. Gleichzeitig verwendet man für den Prozeß einen Mischreaktor und einen Rohrreaktor. Es sei betont, daß die obengenannte Ausbeute an AMS auf der größten Betriebsanlage erreicht wurde.

Den Erfolg, der in den Patenten US 5,254,751 und US 5,530,166 erreicht wird, erhält man im Patent US 4,358,618 bei einem Cumolgehalt von bis zu 15 Gew.-% und äquimolarem Verhältnis von Phenol zu Aceton (Molverhältnis Aceton : Phenol : Cumol = 1 : 1 : 0,23). In diesem Patent benutzt man eine Kombination aus einem Mischreaktor und zwei Rohrreaktoren. Dabei bleibt die Zusammensetzung des Reaktionsmediums in allen Reaktoren konstant, doch verwendet man einen unterschiedlichen Temperaturbereich: im Mischreaktor 50 - 90 °C, in den Rohrreaktoren 120 - 150 °C.

Die Ergebnisse unserer Untersuchungen und der Analyse der Patentdaten zeigen, daß die Menge an Aceton und Cumol, die zusätzlich ins Reaktionsmedium eingeführt wird, für die Selektivität des Prozesses wichtig, aber nicht entscheidend ist. Wie unsere Untersuchungen zeigen, ist die Kombination aller obengenannten Faktoren sehr wichtig, und zwar das optimale molare Verhältnis von Aceton : Phenol : Cumol : Wasser, und eine richtige Auswahl der Reaktoren, für die dieses Verhältnis eingehalten werden muß, d. h. ein Mischreaktor für die Spaltung von CHP und ein Rohrreaktor für die Spaltung von Dicumylperoxid (DCP) und DMPC. Praktisch ist in allen Patenten und funktionierenden Betriebstechnologien die Zusammensetzung des Reaktionsmediums, das durch das optimale Molverhältnis von Aceton : Phenol : Cumol : Wasser charakterisiert wird, in den Durchmischungs- und Rohrreaktoren konstant.

Wir haben jedoch überraschenderweise festgestellt, daß sich die Zusammensetzung des Reaktionsmediums in den obengenannten Reaktoren deutlich voneinander unterscheiden muß, um eine hohe Selektivität und gleichzeitig eine minimale Menge an Nebenprodukten zu erreichen (s. Beispiele 2 - 9).

Im praktischen Prozeß der Herstellung von Phenol und Aceton ist nicht nur die Selektivität des Prozesses äußerst wichtig, sondern auch die Menge der gebildeten Nebenprodukte wie Mesityloxid (MO) und Hydroxyaceton (HA), die die Auftrennung der Produkte erschweren und den Energieaufwand bedeutend erhöhen.

Bisher nahm man in den obigen Patenten auf diesen Umstand keine Rücksicht. Wie unsere Forschungsarbeiten gezeigt haben (s. Tabelle 1), stimmen die optimalen Bedingungen für die Ausbeute an AMS und für die Minimierung der Menge der obengenannten Nebenprodukte nicht überein. So haben wir experimentell festgestellt, daß eine Temperatursteigerung in dem Temperaturbereich, der im Patent US 4,358,618 empfohlen war, in der äquimolaren Mischung von Phenol-Aceton, die 15 Gew.-% Cumol enthält, ein unerwünschtes Ansteigen des Gehalts von MO auf bis zu 750 ppm hervorruft. Das bedeutet, daß einerseits in allen Prozessen bei der Temperatursteigerung eine Verbesserung der Ausbeute an AMS erreicht wird, und daß anderseits die Konzentration an MO bedeutend vergrößert wird (s. Tabelle 1). Da in allen Prozessen strenge Anforderungen an den MO-Gehalt im Phenol (<10 - 15 ppm) gestellt werden, ist bezüglich der Optimierung des Prozesses nicht nur der Gesichtspunkt der Ausbeute an AMS, sondern auch der Gesichtspunkt der Minimierung der Anteile der obengenannten Nebenprodukte und Beimischungen äußerst wichtig.

Tabelle 1

|  | Temperatur, °C | | |
|---|---|---|---|
| Die Kennziffern | 100 | 120 | 140 |
| Ausbeute an AMS[*], % d. Th. | < 70 | 73 | 78 |
| Mesityloxid (MO), ppm | 124 | 319 | 751 |

[*] Die Ausbeute an AMS wird aus Fig. 1 des Patents US 4,358,618 entnommen.

In einem Prozeß mit hohem Acetongehalt im Reaktionsmedium, wie in den Patenten US 2 663 735 und US 1 109 297, erreicht man eine noch höhere Konzentration an MO in den Spaltungsprodukten (1200 ppm). Die vorhandenen großen Mengen an Nebenprodukten führen zu einem bedeutenden Energieaufwand im Prozeß.

Auch gemäß US-P 5,530,166 wird mit einem hohen Überschuß an Aceton (Molverhältnis Aceton:Phenol 1,5: 1) gearbeitet, wobei das Reaktionsmedium bis zu 20 Gew.-% Cumol enthalten kann.

Die DE 19 15 480 A beschreibt die Verwendung von technischem Cumolhydroperoxid, welches mindestens 70 Gew.-% Cumolhydroperoxid enthält. Der Rest von bis zu 30 % besteht aus Cumol und 4 bis 10 % sonstige Nebenprodukte wie DMPC, ACP und DCP. Wesentlich ist, daß das Reaktionsgemisch durch Zusatz von Aceton zur Reaktionsmischung auf einen Acetongehalt von mindestens 37 bis 48 Gew.-% gebracht werden muß, um unerwünschte Nebenreaktionen zu vermeiden. Dies hat zur Folge, daß aus den Reaktionsprodukten zur Gewinnung von Phenol zunächst unter erheblichem Energieaufwand dieser Überschuß an Aceton abgedampft werden muß und das Gesamtreaktionsvolumen entsprechend ansteigt und somit eine größerer apparativer Aufwand getrieben werden muß.

In allen obenerwähnten Stufen des Prozesses von der Oxidation bis zur Isolierung der Endprodukte, treten chemische Verluste der Endprodukte auf, während etwa 75 % des Energieaufwands des ganzen Prozesses auf die Stufe der Rektifikation der Spaltungsprodukte von CHP entfallen.

Zusätzlich wird eine erhebliche Menge Energie benötigt, um das in der Cumol-Oxidationsstufe hergestellte verdünnte CHP auf den für die Zufuhr zur CHP-Spaltungsstufe gewünschten Gehalt aufzukonzentrieren. Da der Umsatz von Cumol in der Oxidationsstufe in den Betriebsprozessen 15 - 35 % beträgt, weil die Selektivität der Oxidationsreaktion oberhalb 35 % Cumolumsatz stark abnimmt, werden die erhaltenen Oxidationsprodukte aufkonzentriert. Das Aufkonzentrieren wird mehrstufig durchgeführt, damit der Restgehalt an Cumol im erhaltenen technischen CHP von 1 - 2 bis zu 10 - 15 Gew.-% beträgt. Dabei entsteht durch den nötigen niedrigen Umsatz an Cumol in der Oxidationsstufe ein zusätzlicher Energieaufwand zur Abtrennung des nicht umgesetzten Cumols aus dem CHP zur nachfolgenden Rückführung von Cumol in die Oxidationsstufe.

Berechnungen und Betriebserfahrung zeigen, daß man 3,87 t Cumol abdestillieren muß, um bei einem Cumolumsatz von 20 % pro Durchgang einen vollständigen Umsatz von 1 t Cumol zu erreichen. Der Dampfverbrauch beträgt dabei 1,2 t / 1 t Phenol und der Cumolgehalt im technischen CHP 1 - 2 %.

Wir haben experimentell festgestellt, daß auf dieser Stufe neben dem Hauptdampfaufwand auch Nebenreaktionen von CHP zu DMPC und zu Acetophenon (ACP) auftreten. Die Menge dieser Nebenprodukte steigt auf 20 % bei einem Restgehalt an Cumol von 1 - 2 Gew.-% im technischen CHP. Die Vergrößerung des Cumolgehalts im technischen CHP bis auf 10 - 15 Gew.% verringert die chemischen Verluste bis auf 8 - 10 %. Die durchgeführte Untersuchung des Einflusses von Cumol auf die Stabilität von CHP hat gezeigt, daß es möglich ist, die thermische Spaltung von CHP bei einer Konzentration von Cumol über 20 Gew.-% im technischen CHP vollständig auszuschließen.

Wie unsere Forschungen und Berechnungen gezeigt haben, sinkt der Energieaufwand in der Aufkonzentrierungsstufe auf etwa 70 % ab und beträgt 0,95 t Dampf / t Phenol, wenn der Cumolgehalt im technischen CHP auf bis zu 25 Gew.-% ansteigt. Gleichzeitig werden die chemischen Verluste an CHP praktisch vollständig unterdrückt (s. Tabelle 2).

Tabelle 2

| Der Gehalt von CHP und Cumol im technischen CHP, Gew.-% | | Zunahme des Gehaltes an DMPC und ACP beim Aufkonzentrieren der Oxidations-produkte in % vom Gemisch | Dampfaufwand beim Aufkonzentrieren der Oxidationsprodukte in t Dampf / t Phenol |
|---|---|---|---|
| CHP | Cumol | | |
| 93,0 | 1,0 | 20 | 1,20 |
| 83,0 | 10,0 | 10 | 1,12 |
| 73,0 | 25,0 | < 0,5 | 0,95 |

Die Vergrößerung des Gehalts an nicht abdestilliertem Cumol im technischen CHP vermindert die chemischen Verluste und den Energieaufwand in dieser Stufe, erschwert aber die Durchführung der homogenen sauren Spaltung von CHP. Deshalb wurde von den Forschern und Betreibern dieses Prozesses die Verwendung einer Cumolkonzentration im technischen CHP von über 10 - 12 Gew.-% nicht empfohlen, obwohl in den Patenten Cumolgehalte bis 20 Gew.-% genannt wurden.

Das Schlüsselproblem der Verwendung hoher Cumolkonzentrationen in der Stufe der sauren Spaltung von CHP ist nämlich die sichere Durchführung dieser Stufe. Ohne Lösung dieses Problems führt die Durchführung des Prozesses in einem Medium mit erhöhtem Cumolgehalt zu den Folgen:

Akkumulation von nicht gespaltenem CHP in den Reaktoren und

Explosion, sofern die Wärmeentwicklung bei der Spaltung des CHP von 1600 kJ / kg (380 kcal / kg) einer Temperatursteigerung auf 700 °C äquivalent ist.

Das zweite zu lösende Problem ist eine bedeutende Erhöhung des Energieaufwands in der Stufe der Rektifikation der Spaltungsprodukte, wenn der Cumolgehalt in diesem Produktgemisch ansteigt.

Die von uns durchgeführten Analysen und Berechnungen zeigen, daß bei dem Prozeß gemäß dem Stand der Technik die Verringerung des Energieaufwands durch Nichtentfernen des Cumols in der Stufe des Aufkonzentrierens von CHP bedeutend überstiegen wird durch den Energie- und Betriebsaufwand zur Trennung der Spaltungsprodukte und Rückführung des erst am Ende des Prozesses abgetrennten Cumols in die Oxidationsstufe. Das gesamte Cumol muß dabei durch alle Stufen des Prozesses geführt werden und erschwert die nachfolgende Reinigung des Phenols auf Verunreinigungsgehalte von einigen ppm erheblich.

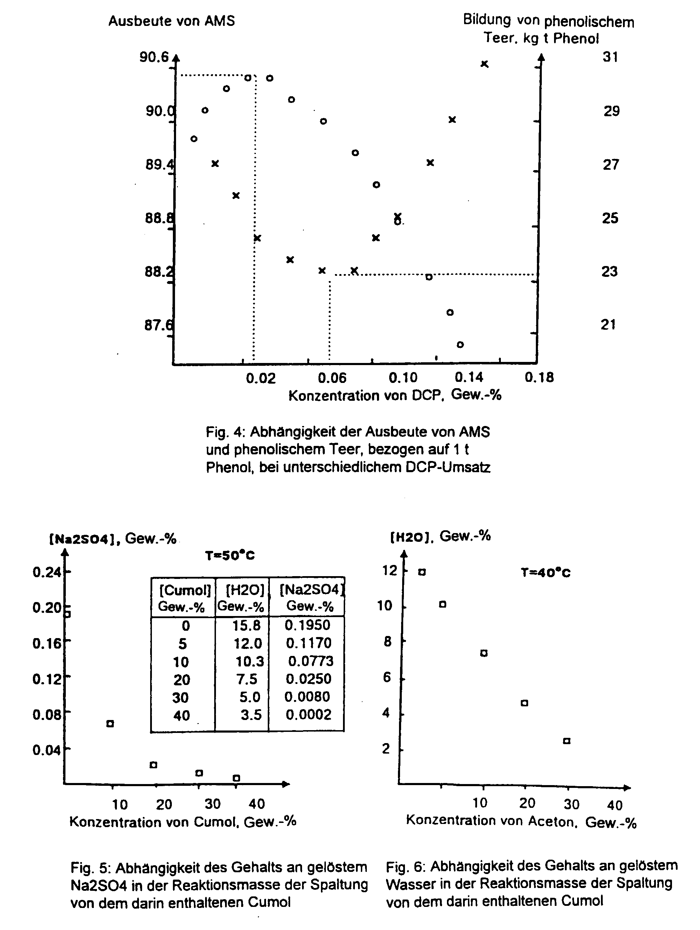

In dem von uns ausgearbeiteten komplexen Verfahren gelingt es, die obengenannten Nachteile zu vermeiden, und einen Energiegewinn für den ganzen Prozeß zu erhalten. Dabei wird die Sicherheit in der Stufe der Spaltung erhöht unter gleichzeitiger Erhöhung der Selektivität und Herabsetzung der Menge der Nebenprodukte. Außerdem gelingt es, die Salze in der Stufe der Neutralisation bedeutend zu verringern. Diese Vorteile ergeben sich aus unseren Laboruntersuchungen, die in den Beispielen 2 - 9 und in Fig. 4 demonstriert werden.

Wie unsere Untersuchungen gezeigt haben, sind die Reaktionen der Umwandlung von Dicumylperoxid (DCP) und DMPC und die Spaltung von CHP in Reaktionsmedien unterschiedlicher Zusammensetzung durchzuführen. Dadurch wird die höhere Selektivität und der höhere Umsatz von DCP erreicht.

Erfindungsgemäß wird die Spaltung, von Dicumylperoxid und Dimethylphenylcarbinol im Rohrreaktor in einem Reaktionsmedium mit gesenktem Gehalt an Aceton durchgeführt wird, wobei der gesenkte Gehalt von Aceton entweder durch Entfernung eines Teils des Acetons, oder mit Hilfe der Zufuhr einer zusätzlichen Menge Cumol- und Wasserfraktion in die Spaltungsprodukte, oder durch Verwendung beider Verfahren erreicht wird.

So wird im äquimolaren Gemisch von Phenol-Aceton, das bis zu 15 Gew.-% Cumol enthält, die optimale Selektivität bei einem Restgehalt an DCP von 0,5 Gew.-% erreicht, was einem Gesamtverlust von 0,5 % abs. der Endprodukte - Phenol, Aceton und AMS- entspricht.

In dem von uns ausgearbeiteten Verfahren gelingt es mit dem Molverhältnis Phenol : Aceton : Cumol = 1 : (1 - 0,77) : (0,35 - 0,87) im Reaktionsmedium die besten Ergebnisse in Hinblick auf die Ausbeute an AMS und den Cumolverbrauch, bezogen auf 1 t Phenol, sowie die Bildung von phenolischem Teer bei einem Restgehalt an DCP von 0,06 - 0,09 Gew.-% zu erreichen, d. h. bei nahezu 100 % Umsatz des DCP, was in anderen Patenten nicht erreicht wird. Gleichzeitig haben wir festgestellt, daß die minimale Bildung von phenolischem Teer und entsprechend die beste Ausnutzung von Cumol bei nicht maximaler Ausbeute an AMS erreicht wird (s. Fig. 4).

Figur 4 zeigt, daß ein Vergleich der Effektivität von verschiedenen Technologien anhand der Ausbeute an AMS nicht ganz korrekt ist, und daß es richtiger ist, die Effektivität der Prozesse anhand des Cumolverbrauchs, bezogen auf das Phenol, zu vergleichen

Die Ergebnisse unserer Untersuchungen, die in Fig. 5 und 6 graphisch dargestellt sind, zeigen, daß die Menge des in der Reaktionsmasse der Spaltung (RMS) gelösten $Na_2SO_4$ von 2000 ppm ohne Cumol auf bis zu 2 ppm bei einem

Cumolgehalt von 40 Gew.-% in der RMS herabgesetzt wird. Diesen Effekt erreicht man in der erfindungsgemäßen Technologie durch die Veränderung der Zusammensetzung des Reaktionsmediums in der Umwandlungsstufe des DCP und durch zusätzliche Einführung von Cumol in der Neutralisationsstufe. Gleichzeitig wird unter diesen Bedingungen die Menge des in der RMS gelösten Wassers von 12 % auf bis zu 3,5 Gew.-% herabgesetzt.

Im Ergebnis löst das erfindungsgemäße Verfahren durch die veränderliche Zusammensetzung des Reaktionsmediums einige technische Probleme:

1. Die effektive Entfernung der Natriumsalze ($Na_2SO_4$, $NaHSO_4$, Na-Phenolate) aus den Produkten, die der Trennung zugeführt werden, was in anderen Technologien nicht erreicht wird.

2. Die Herabsetzung des Energieaufwandes durch Verminderung des Gehaltes an Wasser in der RMS, dessen Verdunstungswärme (2255 kJ / kg (539 kcal / kg)) bedeutend höher ist als die von Cumol (326 kJ / kg (78 kcal / kg)).

3. Die sichere Spaltung von CHP wird gewährleistet.

4. Die hohe Selektivität im Reaktor der Umwandlung von DCP und DMPC wird gewährleistet, was in anderen Technologien nicht erreicht wird.

5. Der Anfall der Nebenprodukte Mesityloxid und Hydroxyaceton wird herabgesetzt (s. Tabelle 3).

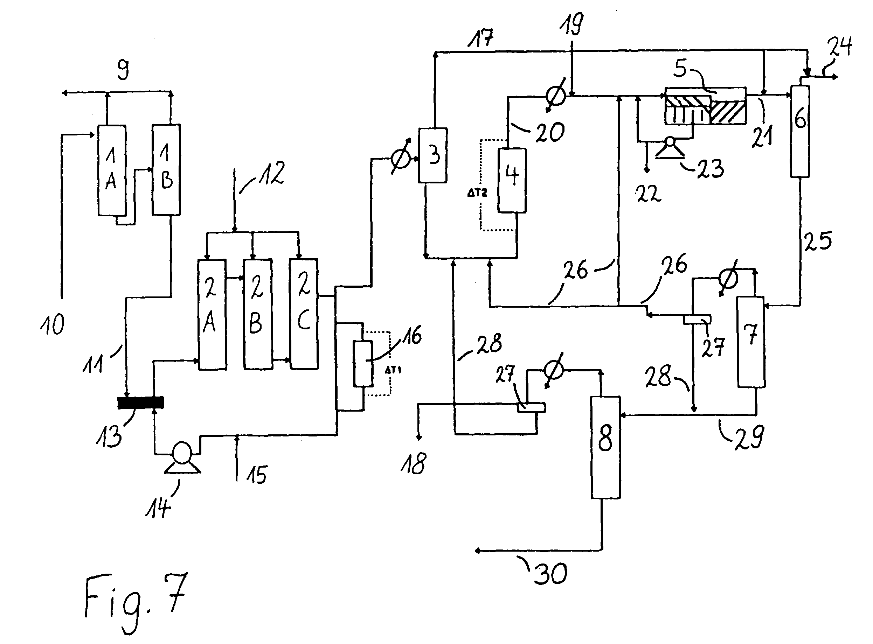

Ein prinzipielles Schema einer besonders bevorzugten Anordnung zur Ausführung des verbesserten Prozesses ist in Fig. 7 dargestellt und wird im folgenden beschrieben.

Die Oxidationsprodukte werden über die Zuleitung 10 in den Apparat 1 A gegeben, wo die Verdampfung eines Teils des Cumols, der durch Leitung 9 in die Oxidation zurückgeführt wird, durch die in den Oxidationsprodukten enthaltene Wärme stattfindet. Das weitere Aufkonzentrieren wird in einem oder zwei aufeinander folgenden Apparaten 1 B, C (der zweite Apparat 1 C ist im Schema nicht gezeigt) durch von außen in einer solchen Menge zugeführte Wärme durchgeführt, daß der Cumolgehalt im erhaltenen technischen CHP 21 % übersteigt, aber weniger als 30 Gew.-% beträgt.

Als Destillationsausrüstung für die Oxidationsprodukte können die üblicherweise verwendeten Standardapparaturen (Vakuumverdampfer mit außen- oder innenliegenden Umlaufverdampfern, Dünnschichtverdampfer mit Filmkondensator u. s. w.) verwendet werden. Unabhängig vom Typ des Verdampfers wird ein Cumolgehalt im Sumpfprodukt von 26 - 28 Gew.-% bevorzugt. Das erhaltene technische CHP hat folgende Zusammensetzung (Gew.-%):

| CHP | 75 - 64, vorzugsweise 67 - 65, |
|---|---|

(fortgesetzt)

| Cumol | 21 - 30, vorzugsweise 28 - 26, |
| DMPC | 8 - 3, |
| ACP | 1,2 - 0,4, |
| DCP | 0,5 - 0,2. |

Die Konzentrationen von DMPC, ACP und DCP können sich je nach dem Oxidationsgrad, der Temperatur und dem pH des Mediums in den Oxidationsreaktoren stark verändern und sind daher auf die obengenannte Zusammensetzung nicht beschränkt. Die Beschränkung bezieht sich nur auf die Konzentrationen von Cumol und CHP.

Zur Spaltung von CHP wird eine herkömmliche Vorrichtung eingesetzt (s. Fig. 7) die aus nicht weniger als drei Wärmeaustauschern 2 (A, B, C) besteht, die durch das in den Röhren fließende Wasser abgekühlt werden, während sich das CHP und die zirkulierenden Spaltungsprodukte in dem Mantelraum befinden. Das Kühlwasser wird über Leitung 12 und die konzentrierten Oxidationsprodukte über Leitung 11 zugeführt. In dem Mischer 13 werden die Oxidationsprodukte mit den in dem Reaktor 2 zirkulierenden Spaltungsprodukten und dem Katalysator gemischt. Die Spaltung von CHP wird mit Hilfe von Schwefelsäure als Katalysator durchgeführt, deren Konzentration von 200 - 300 ppm durch ein Leitfähigkeitsmeßgerät (nicht gezeigt) automatisch geregelt wird. Die Schwefelsäure wird über Leitung 15 der durch die Pumpe 14 umgepumpten Reaktionsmasse zugeführt. Die Spaltung von CHP wird in dem Medium, das vorzugsweise 28 - 26 Gew.-% Cumol enthält, bei einem Molverhältnis von Phenol : Aceton = 1 : 1 durchgeführt. Die Cumolkonzentration läßt sich in der Vorrichtung von 21 bis 30 Gew.-% variieren.

Die Spaltung von technischem CHP, das die veränderliche Konzentration von Cumol enthält, in der obengenannten Vorrichtung wird je nach Cumolgehalt gemäß folgender Formel durchgeführt:

$$G_{zirk} = \frac{480 \times G_{tCHP}}{\% \ Cumol} \tag{1}$$

wobei $G_{zirk}$ die Menge der zirkulierenden Oxidationsprodukte in t / h,
$G_{tCHP}$ die Menge des der Spaltung zugeführten technischen CHP in t / h,
und % Cumol den Gewichtsanteil Cumol im technischen CHP darstellt.

Die Durchführung des Prozesses der CHP-Spaltung in der oben beschriebenen Weise gewährleistet die Stabilität und Sicherheit bei veränderlichem Cumolgehalt im technischen CHP und entsprechend bei veränderlicher Zusammensetzung des Reaktionsmediums, das durch ein Molverhältnis von Phenol : Aceton : Cumol = 1 : 1 : (0,38 - 0,61 ) charakterisiert ist.

Bei veränderlichem Cumolgehalt im technischen CHP bleibt die Größe des CHP-Umsatzes entsprechend der oben erwähnten Formel (1) konstant und wird über die Größe der Temperaturdifferenz $\Delta T_1$ eines Kalorimeters 16 korrigiert, das einen Minirohrreaktor darstellt, der so wie im Schema gezeigt aufgestellt ist (s. Fig. 7). Der CHP-Umsatz pro Durchgang beträgt 76 - 88 %. Bei einer Abweichung von dem mit der Formel (1) bestimmten Verhältnis korrigiert das Signal $\Delta T_1$ über Klappen die Durchlaufmenge des Kühlwassers in den Reaktoren 2A, 2B und 2C.

Der Zusammenhang zwischen dem Verhältnis der Formel (1) und der korrigierenden Einwirkung auf die Klappen, die die Durchlaufmenge des Kühlwassers in den Reaktoren der CHP-Spaltung je nach $\Delta T_1$ regeln, gewährleistet den Doppelschutz des Prozesses der CHP-Spaltung und minimiert die Bildung des dimeren AMS und komplizierter Phenole in dieser Stufe.

Die Spaltungsprodukte von CHP, die nicht umgesetztes CHP enthalten, kommen in den Verdampfer 3, wo Teile des Acetons durch Ausnutzung der Wärmeentwicklung der Spaltungsreaktion abgetrieben werden, um dessen Konzentration und entsprechend die Menge des aus Aceton im Reaktor 4 der Umwandlung von DCP und DMPC gebildeten MO und HA zu senken. Die Verdampfung von Aceton geschieht adiabatisch vorwiegend unter einem Vakuum von 400 - 666 hPa (300 - 500 Torr) oder bei Normaldruck. Das Aceton wird über Leitung 17 abgeleitet.

Das an Aceton verarmte Produkt kommt aus dem unteren Teil des Verdampfers 3 in den Spaltungsreaktor 4 von DCP und DMPC, der nach dem Prinzip der Rohrreaktoren arbeitet. Die Konzentration von CHP in diesem Strom beträgt 0 %. Durch Abtreiben des Acetons im Verdampfer 3 verläuft die anschließende Spaltung von DCP und die Dehydratation von DMPC im Reaktor 4 in einem Reaktionsmedium, das sich von dem Reaktionsmedium, in dem die Spaltung von CHP im Mischreaktor 2 durchgeführt wird, unterscheidet.

Zur Optimierung der Zusammensetzung des Reaktionsmediums für eine erhöhte Ausbeute an AMS werden die Cumolfraktion (der Strom IV) und das zirkulierende Wasser (der Strom III) in Mengen von 160 und von 1 bis zu 30,4 kg bezogen auf 1 t technisches CHP über die Leitungen 26 bzw. 28 dem Rohrreaktor 4 zugeführt. Im Ergebnis entspricht die Zusammensetzung des Reaktionsmediums nach den durchgeführten Änderungen im Reaktor 4 einem Molverhältnis von Phenol :

Aceton : Cumol = 1 : (1 - 0,77) : (0,35 - 0,87).

Die Spaltung von DCP und DMPC in dem Rohrreaktor 4 wird je nach der Menge des zugeführten Cumols und des Wassers bei Temperaturen von 150 bis 168 °C durchgeführt, wie in den Beispielen 2 - 9 beschrieben.

Die Steuerung der Spaltung von DCP und DMPC wird durch das Verhältnis $\Delta T_1 / \Delta T_2$ vorgenommen, das in der Vorrichtung im Bereich von 1,5 bis 21,4 gehalten wird.

Die Größe $\Delta T_1$ stellt die Temperaturdifferenz der Spaltungsprodukte am Eintritt und Austritt im Minireaktor (dem Kalorimeter 16) dar. Die Größe $\Delta T_2$ stellt die Temperaturdifferenz am Eintritt und Austritt im Reaktor 4 der Umwandlung von DCP dar. Die Größe des Verhältnisses muß im Bereich von 1,5 bis 21,4 gehalten werden. Das beste Verhältnis ist 3 - 8. Diese Bedingungen ermöglichen die sichere Durchführung der Spaltung von CHP und eine selektive Umwandlung von DCP bei einem Umsatz des letzteren von über 97 %, was in den anderen Prozessen nicht erreicht wird.

Die Spaltungsprodukte werden nach dem Austritt aus dem Reaktor 4 bis auf eine Temperatur von 30 - 50 °C abgekühlt. Dabei können über Leitung 26 bis zu 255, vorzugsweise bis zu 160 kg / t, bezogen auf 1 t technisches CHP, der Cumolfraktion aus dem oberen Teil der Kolonne 7 und bis zu 20 kg / t Wasser aus einem Separator 27' vom Kopf der Kolonne 8 zugeführt werden.

Die Spaltungsprodukte enthalten noch Schwefelsäure und werden zu deren Entfernung über Leitung 20 dem Neutralisator 5 zugeführt. Die Neutralisation der Schwefelsäure wird durch Zufuhr von alkalischen Agenzien wie NaOH, $Na_2CO_3$ und Na-Phenolate über Leitung 19 verwirklicht. Dabei scheidet sich in der Neutralisationsstufe Natriumsulfat als konzentrierte, wäßrige Phase am Boden der Vorrichtung 5 ab, die über die Pumpe 23 und die Leitung 22 durch Dekantieren entfernt wird. Die durch Zufuhr der Cumolfraktion zu den Spaltungsprodukten erreichte Veränderung der Zusammensetzung des Reaktionsmediums ermöglicht es, die bei der Neutralisation gebildeten Salze in 2 - 3-fach kürzerer Zeit als beim Stand der Technik und mit hoher Wirksamkeit (mehr als 95 %) von der organischen Phase abzutrennen. Die Salzkonzentration in der über Leitung 21 weitergeführten organischen Phase (Strom VI) liegt unter 10 - 20 ppm.

In der Stufe der Neutralisation der Schwefelsäure beträgt der Gesamtgehalt an Cumol und α—Methylstyrol in den Spaltungsprodukten durch die Zufuhr der Kohlenwasserstofffraktion 40 Gew.-%, der Wassergehalt 3,5 Gew.-%, und die Konzentration der Salze in den Spaltungsprodukten nach der Stufe der Neutralisation der Schwefelsäure 3 bis 20 ppm.

Es sei betont, daß in allen existierenden Technologien die Abtrennung von Salzen viel Zeit (von 1,5 bis zu 24 h) in Anspruch nimmt. Das zwingt dazu, sperrige Ausrüstungen mit großem Rauminhalt für das Abstehenlassen der Salze zu verwenden. Trotzdem übersteigt die Wirksamkeit der Abtrennung der Salze 90 % nicht. Für eine standfeste und sichere Funktion der Wärmeaustauscher der Trennungskolonnen müssen deshalb teure Koaleszenzfilter mit spezieller Konstruktion installiert werden. Diese senken den Salzgehalt in den Spaltungsprodukten auf 10 bis 20 ppm, was erfindungsgemäß ohne Verwendung der Filter erreicht wird.

Das Aceton wird, gegebenenfalls nach Zufuhr des im Verdampfer 3 abgetrennten Acetons über die Leitung 17, in Kolonne 6 von den Spaltungsprodukten abgetrennt und über Leitung 24 der weiteren Verarbeitung zugeführt. Die von dem Aceton abgetrennten Spaltungsprodukte (Strom VIII) werden als Sumpfprodukt der Kolonne 6 entnommem und über Leitung 25 der weiteren Rektifikation zugeführt.

Die Abtrennung von Cumol aus den Spaltungsprodukten wird in den zwei nacheinander aufgestellten Kolonnen 7 und 8 durchgeführt. In der Kolonne 7 scheidet sich als Kopfprodukt die Cumol-haltige Fraktion ab, die nicht mehr als 1 % AMS und 0,3 Gew.-% Phenol enthält. Über Kopf dieser Kolonne wird auch praktisch das gesamte Wasser abgeführt, das sich dann von der Cumolfraktion im Separator 27 abtrennt, und der Kolonne 8 zugeführt wird. Als Sumpfprodukt der Kolonne 7 wird das Rohphenol über die Leitung 29 in die Kolonne 8 geführt. Als Kopfprodukt der Kolonne 8 wird über die Leitung 18 und den Separator 27' eine Fraktion Cumol - AMS (Strom IX) abgenommen, die auf bekannte Weise weiter verarbeitet wird. Als Sumpfprodukt der Kolonne 8 wird über die Leitung 30 ein Rohphenol, das praktisch kein Cumol und AMS enthält, abgenommen und auf bekannte Weise weiter verarbeitet.

Über Kopf der Kolonne 8 wird das gesamte Wasser abgenommen, das sich vom Cumol und AMS im Separator 27' abtrennt, und es wird dann dem Reaktor 4 der Umwandlung von DCP und DMPC zugeführt. Dieses Verfahren ermöglicht es, den Bedarf an frischem Wasser im Prozeß um das 2 - 3-fache und entsprechend die Menge des Abwassers proportional zu senken. Die Gesamtverminderung des Energieaufwands in dem im Schema dargestellten erfindungsgemäßen Verfahren unter Verwendung von zwei Kolonnen ist durch die Herabsetzung der Löslichkeit von Wasser in den Spaltungsprodukten von 10 - 12 auf bis zu 3,0 - 3,5 Gew.-% bei der von uns verwendeten Zusammensetzung der Spaltungsprodukte, die der Trennung zugeführt werden, bedingt.

Die Gesamtverminderung des im Prozeß verbrauchten Dampfes beträgt 0,4 - 0,6 t/t Phenol durch die Einsparung der Wärme auf den Stufen des Aufkonzentrierens der Oxidationsprodukte und der Trennung der Spaltungsprodukte.

Die besonderen Unterschiede des von uns ausgearbeiteten Prozesses zu denjenigen in den Patenten RU 1,361,937, US 2,663,735, RO 1,563,181, US 5,254,751, US 5,530,166, US 4,358,618 bestehen in Folgendem:

1. Die Stufe des Aufkonzentrierens von CHP wird bis zu einem Restgehalt von Cumol von 21 bis zu 30 Gew.-%, vorzugsweise 26 - 28 Gew.-% durchgeführt.

2. Die Spaltung von CHP und die Spaltung von DCP wird in Mischreaktoren bzw. Rohrreaktoren bei entsprechend unterschiedlichen Zusammensetzungen des Reaktionsmediums in den obengenannten Reaktoren durchgeführt, während die Zusammensetzung des Reaktionsmediums in allen anderen Technologien konstant gehalten wird.

3. Die Spaltung von DCP und DMPC im Rohrreaktor wird unter folgenden Bedingungen durchgeführt:

a) bei gesenktem Acetongehalt im Reaktionsmedium, der durch die vorläufige Entfernung eines Teils des Acetons und/oder die zusätzliche Zufuhr der Cumol- und Wasserfraktion erreicht wird,

b) bei Temperaturen, die das Reaktionsgleichgewicht der Umwandlung von DMPC zur Seite des AMS verschieben und die nahe an 100 % Umsatz erreichen lassen. In den bisherigen Technologien entspricht das Verhältnis von Phenol : Aceton im Reaktionsmedium 1 : 1 bis zu 1 : 1,5, d. h. dort wird eine zusätzliche Menge Aceton zugeführt. Das von uns verwendete Verfahren der Verminderung des Acetongehalts im Reaktionsmedium wurde früher noch nicht benutzt, ebenso wie das Verfahren der gleichzeitigen Zufuhr von Wasser und der Cumolfraktion in den Spaltungsreaktor von DCP und DMPC. Die Verbindung der erwähnten Verfahren ermöglicht die hohe Selektivität wobei praktisch nahe an 100 % Umsatz erreicht werden, was in anderen Technologien nicht erreicht wurde (die Selektivität ist nicht mehr als 90 %).

4. Die Steuerung des Prozesses der Spaltung von CHP und DCP / DMPC geschieht durch das Verhältnis $\Delta T_1$ / $\Delta T_2$, wobei $\Delta T_2$ die Temperaturdifferenz zwischen Eintritt und Austritt im Spaltungsreaktor von DCP und DMPC ist und $\Delta T_1$ die Temperaturdifferenz des Kalorimeters in der Vorrichtung zur Spaltung von CHP ist, wodurch die erforderliche Sicherheit des Prozesses und seine hohe Selektivität gleichzeitig erzielt werden. In den bisherigen Technologien wird für die Gewährleistung der Sicherheit des Prozesses nur das Verfahren des Kalorimeters ($\Delta T_1$) verwendet.

5. Auf der Stufe der Neutralisation wird für die effektive Entfernung der dort gebildeten Salze die zusätzliche Menge Cumolfraktion zugegeben. Dazu gibt es keine Analogien.

6. Der Wassergehalt der Spaltungsprodukte nach der Stufe der Neutralisation übersteigt 5 Gew.-% nicht, was den Energieaufwand des Prozesses vermindert. In den bisherigen Technologien beträgt der Wassergehalt 6 - 12 Gew.-%.

7. Die in den Spaltungsreaktor von DCP und DMPC und in der Stufe der Entfernung von Salzen zugeführte Cumolfraktion wird durch die verwendete Kolonne abgetrennt und auf diese Stufen in den Prozeß zurückgeführt.

8. Im Prozeß wird der Bedarf an frischem Wasser und entsprechend die Ausgaben für die Abwasserreinigung gesenkt.

Die gegebenen Vorteile und Unterschiede der erfindungsgemäßen Technologie sind in den Beispielen 2 - 9 veranschaulicht. Die Beispiele sind in der Tabelle 3 zusammengestellt.

**Beispiel 1 (Vergleichsbeispiel):**

Die Oxidationsprodukte von folgender Zusammensetzung:

| Zusammensetzung der Oxidations-produkte | Benennung der Stoffe | | | | | Insgesamt |
|---|---|---|---|---|---|---|
| | Cumol | CHP | DMPC | ACP | DCP | |
| Gew.-% | 71,536 | 26,7 | 1,436 | 0,239 | 0,089 | 100 |
| t / h | 147,056 | 54,887 | 2,952 | 0,491 | 0,183 | 205,569 |

(fortgesetzt)

| Zusammensetzung der Oxidations-produkte | Benennung der Stoffe | | | | | Insgesamt |
|---|---|---|---|---|---|---|
| | Cumol | CHP | DMPC | ACP | DCP | |
| Selektivität, mol-% | | | | | | 93,17 |

was der erreichten Gesamtselektivität 93,17 mol-% entspricht, werden der Stufe des Aufkonzentrierens zugeführt, um das technische CHP zu erhalten. Nach dem Aufkonzentrieren hat das technische CHP folgende Zusammensetzung:

| Zusammensetzung des technischen CHP | Benennung der Stoffe | | | | | Insgesamt |
|---|---|---|---|---|---|---|
| | Cumol | CHP | DMCP | ACP | DCP | |
| Gew.-% | 12,31 | 81,9 | 4,7 | 0,8 | 0,3 | 100 |
| t / h | 6,2184 | 41,5314 | 2,3762 | 0,4044 | 0,1517 | 50,502 |
| Selektivität, mol-% | | | | | | 92,7 |

was der erreichten Gesamtselektivität über zwei Stufen - Oxidation und Aufkonzentrieren - von 92,7 mol-% entspricht. Der Verlust der Selektivität durch Teilspaltung von CHP zu DMPC und ACP in der Stufe des Aufkonzentrierens beträgt 0,47 % (abs.). Der Dampfaufwand in der Stufe des Aufkonzentrierens beträgt 0,69 t, bezogen auf 100 % CHP, und 1,12 t, bezogen auf 1 t erhaltenes Phenol.

Das in der Menge von 50,502 t/h erhaltene technische CHP wird in die Stufe der Spaltung geführt. Die Spaltung verläuft in Anwesenheit von 200 - 300 ppm $H_2SO_4$ in einem Reaktionsmedium, dessen Zusammensetzung von den erhaltenen Spaltungsprodukten, CHP und dem zusätzlich zugeführten Aceton bestimmt wird.

Die Spaltung wird laut US 5,530,166 (Beispiel 2) im Reaktorblock durchgeführt, der aus den drei hintereinander aufgestellten Reaktoren besteht, die nach dem Prinzip der Mischreaktoren arbeiten, in denen die Durchmischung durch Zirkulation der Spaltungsprodukte entsteht.

Die aus dem letzten Spaltungsreaktor von CHP austretenden Spaltungsprodukte kommen in den Spaltungsreaktor von DCP, der nach dem Prinzip der Rohrreaktoren arbeitet.

Dem Reaktionsmedium, in dem die Spaltung von CHP durchgeführt wird, wird Aceton gemäß dem angemeldeten Verhältnis in einer Menge von 6025 kg / h zugeführt.

Das Reaktionsmedium hat nach der Zugabe der zusätzlichen Menge Aceton eine Zusammensetzung, die durch das Molverhältnis von Phenol : Aceton : Cumol charakterisiert wird, das im gegebenen konkreten Beispiel 1 : 1,36 : 0,2 ist.

In den Reaktoren der Spaltung von CHP und der Spaltung von DCP wird dieselbe Zusammensetzung des Reaktionsmediums eingehalten.

Die Temperatur im Spaltungsreaktor von DCP beträgt 90 - 100 °C, die Konzentration von Wasser ist 1,38 - 1,7 Gew.-%. Im gegebenen Beispiel ist sie 1,5 Gew.-%.

Das zusätzlich in der Stufe der Spaltung von CHP ins Reaktionsmedium eingeführte Aceton wird im Verdampfer, der nach dem Spaltungsreaktor von DCP aufgestellt ist, abgezogen. Das im Verdampfer abgetriebene und im Kühler kondensierte Aceton wird in die Stufe der Spaltung von CHP in den Prozeß zurückgeführt.

Um die Verluste der Endprodukte Phenol und AMS zu vermindern, wird Ammoniak-Lösung in den Verdampfer für die Teilneutralisation der Schwefelsäure gegeben.

Die Kontrolle und Steuerung der Spaltung von CHP wird wegen der Sicherheit der Stufe durch Einhaltung einer bestimmten Temperaturdifferenz zwischen zwei Kalorimetern vorgenommen, die in der Zirkulationsleitung der Spaltungsprodukte und in der Zufuhrleitung der Produkte in den Spaltungsreaktor von DCP installiert sind. Die Konzentration der wichtigsten Beimischungen und Nebenprodukte im Produktstrom nach dem Verdampfer ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 3,17 | 0,33 | 0,34 | 0,8 | 0,50 | 0,24 | 1500 | 300 |

Die Ausbeute AMS beträgt 76 % d. Th. nach der Stufe der Spaltung, die Bildung von phenolischem Teer ist 44 kg / t Phenol.

Die erhaltenen Spaltungsprodukte werden mit Hilfe von NaOH neutralisiert, bis zu einem Wassergehalt von 10 - 12 Gew.-% mit Wasser versetzt und in die Stufe der Abtrennung der Salze geführt. Nach der Abtrennung des größten Teils der Salze beträgt der Gehalt an letzteren in der Reaktionsmasse der Spaltung (RMS) 2 000 ppm.

Die 12 Gew.-% Wasser enthaltende RMS wird auf übliche Weise der Trennung zugeführt, wo die Abtrennung von Aceton, von einem Gemisch aus Cumol-AMS, von Phenol und den Produkten der Teerkondensation vorgenommen wird.

Der Dampfaufwand in der Stufe der Abtrennung von Aceton und dem Gemisch von Cumol-AMS beträgt 2,9 t / t Phenol.

Der Verbrauchskoeffizient Cumol / Phenol beträgt 1318 kg / t in der Stufe der Spaltung.

Als Ergebnis erhält man im Prozeß:

| Wertprodukte, kg / h | Phenol 25470,4 | Aceton 15869,4 | α-Methylstyrol 1616 |
|---|---|---|---|
| Nebenprodukte am Austritt aus der Vorrichtung zur Spaltung von Cumolhydroperoxid, kg / t Phenol | | | "phenolischer Teer" 44 |
| Dampfaufwand, t / t Phenol | bei der Destillation 1,12 | bei der Spaltung 0,07 | bei der Abtrennung von Aceton und AMS/Cumol 2,9 |
| Gesamtaufwandskoeffizient Cumol / Phenol, einschließlich Cracken des "phenolischen Teers", kg/t Phenol | | | 1313 |

## Beispiel 2

Die Produkte der Oxidationsstufe von Cumol, wo die Selektivität 93,17 mol erreicht, deren Zusammensetzung dieselbe ist, wie im Beispiel 1, werden in die Konzentrationsstufe des CHP gegeben. Nach dem Aufkonzentrieren hat das technische CHP folgende Zusammensetzung:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 30,0 | 65,68 | 3,52 | 0,59 | 0,21 | 100 |
| t / h | 18,900 | 41,3784 | 2,2176 | 0,3717 | 0,1323 | 63,0 |
| Gesamtselektivität, mol-% | | | | | | 93,17 |

was einer Gesamtselektivität über zwei Stufen von 93,17 mol-% entspricht und die Abwesenheit der unselektiven Spaltung von CHP in der Stufe seiner Aufkonzentrierung beweist.

Der Dampfaufwand in der Stufe des Aufkonzentrierens beträgt 0,576 t, bezogen auf 1 t 100 % CHP, und 0,933 t, bezogen auf 1 t erhaltenes Phenol.

Das in der Menge von 63 t / h erhaltene technische CHP wird der Spaltungsstufe zugeführt, die so ausgeführt wird, wie es in der obigen Beschreibung des Schemas des Prozesses beschrieben wurde.

Die Spaltung verläuft in einem Reaktionsmedium, in dem ein Molverhältnis von Phenol : Aceton : Cumol = 1 : 1 : 0,61 eingehalten wird.

Die Zufuhr der zirkulierenden Produkte wird laut Formel (1)

$$G_{zirk} = (480*63) / 30 = 1008 \text{ t / h}$$

eingestellt, wobei der Umsatz pro Durchgang konstant gehalten wird. Der Umsatz wird als Differenz zwischen der Konzentration CHP am Eintritt in den Reaktor A nach der Vermischung mit den zirkulierenden Produkten und am Austritt aus dem Reaktor C, bezogen auf die Konzentration des CHP am Eintritt in den Reaktor A, berechnet und beträgt (4,3-0,73) / 4,3*100 = 83 %.

Die Größe des Verhältnisses $\Delta T_2 / \Delta T_1$ = 2,3. Die Temperatur des Prozesses der Spaltung von CHP beträgt 50°C bei einem Verhältnis $\Delta T_2 / \Delta T_1$ = 2,3 und $G_{zirk}$ = 1008 t / h. Sie wird durch Veränderung der Kühlwasserzufuhr in den Wärmeaustauschern der Reaktoren 2 A, B, C erreicht.

Die RMS kommt in den Verdampfer, in dem das Gemisch von Aceton, Cumol, Wasser und Phenol in einer Menge von 4 400 kg / h unter einem Vakuum von 453 hPa (340 Torr) abdestilliert wird.

Dem Strom von den aus dem unteren Teil des Verdampfers 3 austretenden Produkten werden 10 000 kg / h Cumol und 1 000 kg / h Wasser zugeführt. Als Ergebnis der durchgeführten Veränderungen wird das Reaktionsmedium durch ein Molverhältnis von Phenol : Aceton : Cumol = 1 : 0,78 : 0,87 charakterisiert. Die Wasserkonzentration in den Produkten, die in den Spaltungsreaktor von DMPC und DCP kommen, beträgt 2,50 Gew.-%. Der Prozeß der Spaltung von DMPC und DCP verläuft bei einer Temperatur von 168°C. Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,55 | 0,13 | 0,04 | 0,53 | 0,19 | 0,01 | 950 | 70 |

Die Ausbeute an AMS beträgt 90,6 % d. Th. nach der Stufe der Spaltung von DCP und DMPC, die Bildung von phenolischem Teer 24,4 kg / t Phenol.

Die erhaltenen Spaltungsprodukte werden mit Hilfe von NaOH neutralisiert, bis zu einem Wassergehalt von 3,5 Gew.-% entwässert und in die Stufe der Abtrennung der Salze geführt.

Durch Erhöhung des Cumolgehalts in den Spaltungsprodukten bis auf 40 Gew.-% verläuft die Abtrennung der Salze effektiv und am Austritt aus der Neutralisationsstufe 5 beträgt die Konzentration der Salze in der organischen Phase 3 ppm.

Die von Salzen gereinigten Produkte (Strom VI) kommen in die Kolonne 6, wo die Acetonfraktion als Kopfprodukt (Strom VII) abgenommen wird. Das Sumpfprodukt dieser Kolonne kommt in die Kolonne 7, wo die Abtrennung der Cumolfraktion und des Wassers vorgenommen wird, die im Separator getrennt werden. Die Cumolfraktion in einer Menge von 10 000 kg / h kommt in den Reaktor 4 und die Wasserphase in einer Menge von 1 000 kg / h (Strom III) wird aus dem Separator der Kolonne 7 in die Kolonne 8 geleitet, wo die Cumolfraktion und das Wasser als Kopfprodukt abgenommen werden. Nach der Trennung im Separator wird die Fraktion Cumol-AMS zur Hydrierung und das Wasser in die Vorrichtung zur Spaltung von DCP und DMPC geführt.

Der Dampfaufwand in der Stufe der Abtrennung von Aceton und dem Gemisch von Cumol-AMS beträgt 2,46 t / t Phenol.

Der Verbrauchskoeffizient Cumol / Phenol beträgt 1 300 kg / t in der Stufe der Spaltung.

Als Ergebnis erhält man im Prozeß:

| Wertprodukte, kg / h | Phenol 25573,6 | Aceton 15817,5 | α-Methylstyrol 1794 |
|---|---|---|---|
| Nebenprodukte am Austritt aus der Vorrichtung zur Spaltung von Cumolhydroperoxid in kg / t Phenol | | | "phenolischer Teer" 24,4 |
| Dampfaufwand in t / t Phenol | bei der Destillation 0,93 | bei der Spaltung 0,08 | bei der Abtrennung von Aceton und dem AMS-Cumol-Gemisch 2,46 |
| Gesamtaufwandskoeffizient Cumol / Phenol einschließlich der Crackung des "phenolischen Teers" in kg / t Phenol | | | 1302 |

**Beispiel 3:**

Prozeß nach Beispiel 2, wobei das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 28,0 | 67,47 | 3,69 | 0,62 | 0,22 | 100 |
| t / h | 17,1679 | 41,3686 | 2,2625 | 0,3801 | 0,1349 | 61,314 |
| Gesamtselektivität, mol-% | | | | | | 93,10 |

was der erreichten Gesamtselektivität über zwei Stufen von 93,1 Gew.-% entspricht und die minimale Spaltung von CHP in der Stufe seiner Aufkonzentrierung (0,07 % abs.) anzeigt. Der Dampfaufwand in der Stufe des Aufkonzentrierens beträgt 0,58 t, bezogen auf 1 t 100 % CHP, und 0,94 t, bezogen auf 1 t erhaltenes Phenol.

Das in der Menge von 61,314 t / h erhaltene technische CHP wird der Stufe der Spaltung zugeführt, die so wie in der obigen Beschreibung des Schemas des Prozesses beschrieben durchgeführt wird.

Die Spaltung von CHP wird in einem Reaktionsmedium durchgeführt, in dem das Molverhältnis von Phenol : Aceton : Cumol ist 1 : 1 : 0,55 beträgt. Die Zufuhr der zirkulierenden Produkte wird gemäß der Formel (1)

$$G_{zirk} = (480*61,31) / 28 = 1051 \text{ t / h}$$

vorgenommen, so daß der Umsatz CHP bei einer Größe des Verhältnisses $\Delta T_2 / \Delta T_1 = 1,5$ pro Durchgang 76 % beträgt.

Die RMS kommt in den Verdampfer, in dem das Gemisch von Aceton, Cumol, Wasser und Phenol in einer Menge von 4400 kg / h unter einem Vakuum von 460 hPa (345 Torr) abdestilliert wird.

Dem Strom von den aus dem unteren Teil des Verdampfers 3 ausgehenden Produkten werden 6200 kg / h Cumol und 900 kg / h Wasser zugeführt. Als Ergebnis der durchgeführten Veränderungen wird das Reaktionsmedium durch ein Molverhältnis von Phenol : Aceton : Cumol = 1 : 0,78 : 0,70 charakterisiert. Die Wasserkonzentration in den Produkten, die in den Spaltungsreaktor von DMPC und DCP kommen, beträgt 2,75 Gew.-%.

Der Prozeß der Spaltung von DMPC und DCP verläuft bei einer Temperatur von 160°C. Die Konzentration der wichtigsten Beimischungen und Nebenprodukte im Produktstrom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,81 | 0,14 | 0,05 | 0,59 | 0,20 | 0,02 | 920 | 60 |

Die Ausbeute an AMS beträgt 90,3 % d. Th. nach der Stufe der Spaltung von DCP und DMPC, die Bildung von phenolischem Teer 25,4 kg / t Phenol.

Den erhaltenen Spaltungsprodukten wird eine solche Menge Cumolfraktion zugeführt, daß der Gesamtgehalt an Cumol und AMS 40 Gew.-% beträgt. Die Menge Cumolfraktion, die als Kopfprodukt der Kolonne 7 abgetrennt wird, beträgt 8790 kg / h, von denen 6200 kg / h in die Stufe der Spaltung von DCP und DMPC und 2590 kg / h in die Stufe der Neutralisation geführt werden.

Die Neutralisation der Säure und die Abtrennung der Salze von den Produkten der Reaktion wird analog zu Beispiel 2 durchgeführt. Der Gehalt an Salzen in der RMS beträgt 17 ppm.

Der Dampfaufwand in der Stufe der Abtrennung von Aceton und dem Cumol-AMS-Gemisch beträgt 2,46 t / t Phenol.

Der Verbrauchskoeffizient Cumol / Phenol beträgt 1301 kg / t in der Stufe der Spaltung.

Als Ergebnis erhält man im Prozeß:

| Wertprodukte, kg / h | Phenol 25564,5 | Aceton 15812,2 | $\alpha$-Methylstyrol 1825 |
|---|---|---|---|
| Nebenprodukte am Austritt aus der Vorrichtung zur Spaltung von Cumolhydroperoxid in kg / t Phenol | | | "phenolischer Teer" 25,4 |
| Dampfaufwand in t / t Phenol | bei der Destillation 0,94 | bei der Spaltung 0,08 | bei der Abtrennung von Aceton und dem AMS-Cumol-Gemisch 2,46 |
| Gesamtaufwandskoeffizient Cumol / Phenol einschließlich der Crackung des "phenolischen Teers" in kg / t Phenol | | | 1303 |

**Beispiel 4:**

Der Prozeß wird analog zu Beispiel 2 durchgeführt, wobei das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 21,01 | 73,94 | 4,09 | 0,70 | 0,26 | 100 |

(fortgesetzt)

| Zusammensetzung des technischen CHP | Benennung der Stoffe | | | | | Insgesamt |
|---|---|---|---|---|---|---|
| | Cumol | CHP | DMPC | ACP | DCP | |
| t / h | 11,7522 | 41,3591 | 2,2878 | 0,3916 | 0,1453 | 55,936 |
| Gesamtselektivität, mol-% | | | | | | 93,0 |

was einer erreichten Gesamtselektivität über zwei Stufen von 93 mol-% entspricht und einen Verlust an CHP bei seiner Aufkonzentrierung von 0,17 % anzeigt.

Der Dampfaufwand in der Stufe des Aufkonzentrierens beträgt 0,63 t, bezogen auf 1 t 100 % CHP, und 1,027 t, bezogen auf 1 t erhaltenes Phenol.

Das in einer Menge von 55,94 t / h erhaltene technische CHP wird in die Stufe der Spaltung geführt, die wie in der obigen Beschreibung des Schemas des

Prozesses beschrieben durchgeführt wird.

Die Spaltung von CHP wird in einem Reaktionsmedium durchgeführt, in dem ein Molverhältnis von Phenol : Aceton : Cumol = 1 : 1 : 0,38 eingehalten wird.

Die Zufuhr der zirkulierenden Produkte wird gemäß der Formel (1)

$G_{zirk}$ = (480*55,94)/21 = 1278 t / h durchgeführt, so daß der Umsatz CHP bei einer Größe des Verhältnisses $\Delta T_2 / \Delta T_1$ = 3,76 pro Durchgang 88,0 % beträgt.

Die RMS kommt in den Verdampfer, in dem das Gemisch aus Aceton, Cumol, Wasser und Phenol in einer Menge von 4 400 kg / h unter einem Vakuum von 453 hPa (340 Torr) abgetrieben wird. Dem Strom der aus dem unteren Teil des Verdampfers 3 kommenden Produkte werden 8 500 kg / h Cumol und 1 000 kg/h Wasser zugeführt. Als Ergebnis der durchgeführten Veränderungen wird das Reaktionsmedium durch ein Molverhältnis von Phenol : Aceton : Cumol = 1 :

0,77 : 0,61 charakterisiert. Die Wasserkonzentration in den Produkten, die in den Spaltungsreaktor von DMPC und DCP kommen, beträgt 2,8 Gew.-%.

Der Prozeß der Spaltung von DMPC und DCP verläuft bei einer Temperatur von 151°C. Die Konzentration der wichtigsten Beimischungen und Nebenprodukte im Produktstrom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,94 | 0,16 | 0,07 | 0,63 | 0,24 | 0,05 | 890 | 50 |

Die Ausbeute an AMS beträgt 88,6 % d. Th. nach der Stufe der Spaltung von DCP und DMPC, die Bildung von phenolischem Teer 28,1 kg / t Phenol.

Den erhaltenen Spaltungsprodukten wird eine solche Menge Cumolfraktion zugeführt, daß der Gesamtgehalt an Cumol und AMS 40 Gew.-% beträgt. Die Menge Cumolfraktion, die als Kopfprodukt der Kolonne 7 abgetrennt wird, beträgt 14 200 kg / h, von denen 8 500 kg / h in die Stufe der Spaltung von DCP und DMPC und 5 700 kg / h in die Stufe der Neutralisation geführt werden.

Die Neutralisation der Säure und die Abtrennung der Salze von den Produkten der Reaktion wird analog Beispiel 2 durchgeführt. Der Gehalt an Salz in der RMS beträgt 15 ppm.

Der Dampfaufwand in der Stufe der Abtrennung von Aceton und dem Gemisch aus Cumol-AMS beträgt 2,55 t / t Phenol.

Der Verbrauchskoeffizient Cumol / Phenol beträgt 1 303 kg / t in der Stufe der Spaltung.

Als Ergebnis erhält man im Prozeß:

| Wertprodukte, kg / h | Phenol 25548,26 | Aceton 15805,8 | α-Methylstyrol 1814 |
|---|---|---|---|
| Nebenprodukte am Austritt aus der Vorrichtung zur Spaltung von Cumolhydroperoxid in kg / t Phenol | | | "phenolischer Teer" 28,1 |
| Dampfaufwand in t / t Phenol | bei der Destillation 0,93 | bei der Spaltung 0,08 | bei der Abtrennung von Aceton und dem AMS-Cumol-Gemisch 2,55 |

(fortgesetzt)

| Gesamtaufwandskoeffizient Cumol / Phenol einschließlich der Crackung des "phenolischen Teers" in kg / t Phenol | 1304 |
|---|---|

**Beispiel 5:**

Der Prozeß wird analog Beispiel 4 durchgeführt, wobei das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 28,00 | 67,47 | 3,69 | 0,62 | 0,22 | 100 |
| t / h | 17,1679 | 41,3686 | 2,2625 | 0,3801 | 0,1349 | 61,314 |
| Gesamtselektivität, mol-% | | | | | | 93,1 |

Das in einer Menge von 61,31 t / h erhaltene technische CHP wird der Stufe der Spaltung zugeführt, in der im Reaktionsmedium ein Molverhältnis von Phenol:
Aceton : Cumol = 1 : 1 : 0,38 eingehalten wird.
Die Zufuhr der zirkulierenden Produkte wird laut Formel (1)

$$G_{zirk} = (480 \times 61,314) / 28 = 1\ 051\ t / h$$

so eingestellt, daß der Umsatz von CHP bei einer Größe des Verhältnisses $\Delta T_2 / \Delta T_1 = 2,3$ pro Durchgang 76,0 % beträgt.
Die Zusammensetzung des Reaktionsmedium in dem Spaltungsreaktor von DCP und DMPC wird durch ein Molverhältnis von Phenol : Aceton : Cumol =
1 : 1 : 0,53 charakterisiert. Die Wasserkonzentration in den Produkten, die in den Spaltungsreaktor von DMPC und DCP kommen, beträgt 2,8 Gew.-%.
Der Prozeß der Spaltung von DMPC und DCP verläuft bei einer Temperatur von 156°C.
Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,85 | 0,18 | 0,06 | 0,61 | 0,27 | 0,00 | 910 | 55 |

Die Ausbeute an AMS beträgt 88,31 % d. Th. nach der Stufe der Spaltung von DCP und DMPC, die Bildung von phenolischem Teer 27,7 kg / t Phenol.
Den erhaltenen Spaltungsprodukten wird eine solche Menge Cumolfraktion zugeführt, daß der Gesamtgehalt an Cumol und AMS 40 Gew.-% beträgt. Die Cumolfraktion, die als Kopfprodukt der Kolonne 7 in einer Menge von 10915 kg / h abgetrennt wird, wird nur in die Stufe der Neutralisation gegeben. Die Neutralisation der Säure und die Abtrennung der Salze von den Produkten der Reaktion wird analog Beispiel 2 durchgeführt. Der Gehalt an Salzen in der RMS beträgt 18 ppm.
Der Dampfaufwand in der Stufe der Abtrennung von Aceton und dem Gemisch von Cumol-AMS beträgt 2,46 t / t Phenol.
Der Aufwandskoeffizient Cumol / Phenol beträgt 1302 kg / t in der Stufe der Spaltung.
Als Ergebnis erhält man im Prozeß:

| Wertprodukte in kg / h | Phenol 25553,8 | Aceton 15799,0 | α-Methylstyrol 1784,6 |
|---|---|---|---|
| Nebenprodukte am Austritt aus der Vorrichtung zur Spaltung von Cumolhydroperoxid in kg / t Phenol | | | "phenolischer Teer" 27,7 |

(fortgesetzt)

| Dampfaufwand in t / t Phenol | bei der Destillation 0,94 | bei der Spaltung 0,08 | bei der Abtrennung von Aceton und einem AMS-Cumol-Gemisch 2,46 |
|---|---|---|---|
| Gesamtaufwandskoeffizient Cumol / Phenol einschließlich der Crackung des "phenolischen Teers" in kg / t Phenol | | | 1304 |

**Beispiel 6:**

Produkte der Oxidationsstufe von Cumol, die folgende Zusammensetzung haben:

| Zusammensetzung der Oxidationsprodukte | Benennung der Stoffe | | | | | Insgesamt |
|---|---|---|---|---|---|---|
| | Cumol | CHP | DMPC | ACP | DCP | |
| Gew.-% | 72,374 | 24,2 | 2,931 | 0,382 | 0,113 | 100 |
| t / h | 152,646 | 53,346 | 3,759 | 0,519 | 0,152 | 219,903 |
| Gesamtselektivität, mol-% | | | | | | 86,47 |

was einer erreichten Selektivität von 86,47 mol-% entspricht, werden in die Stufe des Aufkonzentrierens gegeben, um das technische CHP zu erhalten.

Nach dem Aufkonzentrieren hat das technische CHP folgende Zusammensetzung:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 21,0 | 69,2 | 8,35 | 1,12 | 0,33 | 100 |
| t / h | 12,1989 | 40,1983 | 4,8505 | 0,6506 | 0,1917 | 58,09 |
| Gesamtselektivität, mol-% | | | | | | 86,3 |

was einer erreichten Gesamtselektivität von 86,3 mol-% über die zwei Stufen der Oxidation und Aufkonzentrierung entspricht. Der Verlust der Selektivitat beträgt 0,17 % abs. durch Teilspaltung von CHP zu DMPC und ACP in der Stufe seiner Aufkonzentrierung. Das in einer Menge von 58,09 t / h erhaltene technische CHP wird in die Stufe der Spaltung geführt.

Wenn die Spaltung von gegebenem technischen CHP, das 8,35 Gew.-% DMPC enthält, so durchgeführt wird, wie es im Beispiel 1 (Vergleichsbeispiel) beschrieben ist, so wird als Ergebnis der Durchführung des Prozesses die unten dargestellte Konzentration von wichtigen Beimischungen und Nebenprodukten im Strom der Produkte am Austritt aus dem Verdampfer für das Aceton erhalten:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 5,72 | 0,56 | 0,32 | 0,89 | 0,84 | 0,01 | 1500 | 300 |

Die Ausbeute an AMS beträgt 77,8 % d. Th. nach der Stufe der Spaltung von DCP und DMPC, die Bildung von phenolischem Teer 77,6 kg / t Phenol.

Der Dampfaufwand in der Stufe der Abtrennung von Aceton und dem Gemisch aus Cumol-AMS beträgt 3,2 t / t Phenol. Der Verbrauchskoeffizient Cumol / Phenol beträgt 1350 kg / t in der Stufe der Spaltung.

Als Ergebnis erhält man im Prozeß:

| Wertprodukte in kg / h | Phenol 24651,8 | Aceton 15351,8 | $\alpha$-Methylstyrol 3336 |
|---|---|---|---|
| Nebenprodukte am Austritt aus der Vorrichtung zur Spaltung von Cumolhydroperoxid in kg / t Phenol | | | "phenolischer Teer" 44 |

(fortgesetzt)

| Dampfaufwand in t / t Phenol | bei der Destillation 1,12 | bei der Spaltung 0,07 | bei der Abtrennung von Aceton und dem AMS-Cumol-Gemisch 3,2 |
|---|---|---|---|
| Gesamtaufwandskoeffizient Cumol / Phenol einschließlich der Crackung des "phenolischen Teers" in kg / t Phenol | | | 1332 |

Die erfindungsgemäße Durchführung der Spaltung des erhaltenen technischen CHP wird folgendermaßen vorgenommen:

Die Zusammensetzung des Reaktionsmediums der Spaltung des CHP wird durch ein Molverhältnis von Phenol : Aceton : Cumol = 1 : 1 : 0,42 charakterisiert.

Die Zufuhr der zirkulierenden Produkte wird laut Formel (1)

$G_{zirk}$ = (480 x 58,09) / 21 = 1328 t / h so eingestellt, daß der Umsatz CHP bei der Größe des Verhältnisses $\Delta T_2 / \Delta T_1$ = 5,9 pro Durchgang 77,09 % beträgt. Die Temperatur des Prozesses der Spaltung von CHP bei der Größe des Verhältnisses $\Delta T_2 / \Delta T_1$ = 5,9 und $G_{zirk}$ = 1328 t / h von 52°C wird durch Veränderung der Kühlwasserzufuhr in den Wärmeaustauschern der Reaktoren 2 A, B, C eingestellt.

Dem Strom von den aus dem unteren Teil des Verdampfers austretenden Produkten werden 1156 kg / h Wasser zugeführt. Im Spaltungsreaktor von DCP und DMPC wird die Zusammensetzung des Reaktionsmediums durch ein Molverhältnis von Phenol : Aceton : Cumol = 1 : 1 : 0,39 charakterisiert. Die Wasserkonzentration in den Produkten, die in den Spaltungsreaktor von DMPC und DCP kommen, beträgt 3,0 Gew.-%. Der Prozeß der Spaltung von DMPC und DCP verläuft bei einer Temperatur von 168°C.

Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 6,19 | 0,53 | 0,11 | 1,1 | 0,79 | 0,02 | 950 | 70 |

Die Ausbeute an AMS beträgt 85,4 % d. Th. nach der Stufe der Spaltung von DCP und DMPC, die Bildung von phenolischem Teer 60,4 kg / t Phenol.

Den erhaltenen Spaltungsprodukten wird eine solche Menge Cumolfraktion zugeführt, daß der Gesamtgehalt an Cumol 40 Gew.-% beträgt. Die Cumolfraktion, die in einer Menge von 13710 kg / h als Kopfprodukt der Kolonne 7 abgetrennt wird, wird in die Stufe der Neutralisation geführt.

Die Neutralisation der Säure und die Abtrennung der Salze von den Produkten der Reaktion wird analog Beispiel 2 durchgeführt. Der Gehalt an Salzen in der RMS beträgt 8 ppm.

Der Dampfaufwand in der Stufe der Abtrennung von Aceton und dem Gemisch aus Cumol-AMS beträgt 2,8 t / t Phenol.

Der Verbrauchskoeffizient Cumol / Phenol beträgt 1334 kg / t in der Stufe der Spaltung.

Als Ergebnis erhält man im Prozeß:

| Wertprodukte in kg / h | Phenol 24717,8 | Aceton 15364,6 | α-Methylstyrol 3665,6 |
|---|---|---|---|
| Nebenprodukte am Austritt aus der Vorrichtung zur Spaltung von Cumolhydroperoxid in kg / t Phenol | | | "phenolischer Teer" 60,4 |
| Dampfaufwand in t / t Phenol | bei der Destillation 1,03 | bei der Spaltung 0,08 | bei der Abtrennung von Aceton und einem AMS-Cumol-Gemisch 2,8 |
| Gesamtaufwandskoeffizient Cumol / Phenol einschließlich der Crackung des "phenolischen Teers" in kg / t Phenol | | | 1322 |

**Beispiel 7:**

Der Prozeß wird analog Beispiel 3 durchgeführt, wo das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 21,01 | 73,94 | 4,09 | 0,70 | 0,26 | 100 |
| t/h | 17,7522 | 41,3591 | 2,2878 | 0,3916 | 0,1453 | 55,936 |
| Gesamtselektivität, mol-% | | | | | | 93,0 |

Das in einer Menge von 55,94 t / h erhaltene technische CHP wird in die Stufe der Spaltung geführt, bei der im Reaktionsmedium das Molverhältnis von Phenol:

Aceton : Cumol = 1 : 1 : 0,55 beträgt.

Die Zufuhr der zirkulierenden Produkte wird laut Formel (1)

$G_{zirk}$ = (480x55,94) / 2l = 1279 t / h so eingestellt, daß der Umsatz CHP bei der Größe des Verhältnisses $\Delta T_2$ / $\Delta T_1$, = 5 - 7 pro Durchgang von 88 % beträgt.

Die RMS wird in den Verdampfer gegeben, in dem das Gemisch von Aceton, Cumol, Wasser und Phenol in einer Menge 4400 kg / h unter einem Vakuum von 440 hPa (330 Torr) abgetrennt wird.

Dem Strom der aus dem unteren Teil des Verdampfers 3 austretenden Produkte werden 8500 kg / h Cumol und 580 kg / h Wasser zugeführt; Als Ergebnis der durchgeführten Veränderungen wird das Reaktionsmedium durch ein Molverhältnis von Phenol : Aceton : Cumol 1 : 0,77 : 0,61 charakterisiert. Die Wasserkonzentration in den Produkten, die in den Spaltungsreaktor von DMPC und DCP kommen, beträgt 2,1 Gew.-%.

Der Prozeß der Spaltung von DMPC und DCP verläuft bei einer Temperatur von 151°C.

Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,93 | 0,20 | 0,06 | 0,64 | 0,29 | 0,01 | 890 | 50 |

Die Ausbeute an AMS beträgt 87,6 % d. Th. nach der Stufe der Spaltung von DCP und DMPC, die Bildung von phenolischem Teer 28,8 kg / t Phenol.

Den erhaltenen Spaltungsprodukten wird eine solche Menge Cumolfraktion zugeführt, daß der Gesamtgehalt an Cumol 40 Gew.-% beträgt. Die Menge Cumolfraktion, die als Kopfprodukt der Kolonne 7 ausgeschieden wird, beträgt 14100 kg / h, von denen 8500 kg / h in die Stufe der Spaltung von DCP und DMPC und 5600 kg / h in die Stufe der Neutralisation geführt werden.

Die Neutralisation der Säure und die Abtrennung der Salze von den Produkten der Reaktion wird analog Beispiel 2 durchgeführt. Der Gehalt der Salze in der RMS beträgt 17 ppm. Der Dampfaufwand in der Stufe der Abtrennung von Aceton und dem Gemisch aus Cumol-AMS beträgt 1,55 t / t Phenol.

Der Verbrauchskoeffizient Cumol / Phenol beträgt 1305 kg / t in der Stufe der Spaltung.

Als Ergebnis erhält man im Prozeß:

| Wertprodukte in kg / h | Phenol 25545,5 | Aceton 15812,7 | α-Methylstyrol 1794 |
|---|---|---|---|
| Nebenprodukte am Austritt aus der Vorrichtung zur Spaltung von Cumolhydroperoxid in kg / t Phenol | | | "phenolischer Teer" 28,8 |
| Dampfaufwand in t / t Phenol | bei der Destillation 1,03 | bei der Spaltung 0,08 | bei der Abtrennung von Aceton und einem AMS-Cumol-Gemisch 2,55 |
| Gesamtaufwandskoeffizient Cumol / Phenol einschließlich der Crackung des "phenolischen Teers" in kg / t Phenol | | | 1305 |

**Beispiel 8:**

Der Prozeß wird analog zu Beispiel 3 durchgeführt, wobei das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 21,01 | 73,94 | 4,09 | 0,70 | 0,26 | 100 |
| t / h | 17,7522 | 41,3591 | 2,2878 | 0,3916 | 0,1453 | 55,936 |
| Gesamtselektivität, mol-% | | | | | | 93,0 |

Das in einer Menge von 55,94 t / h erhaltene technische CHP wird in die Stufe der Spaltung geführt, die in einem Reaktionsmedium durchgeführt wird, in dem man ein Molverhältnis von Phenol : Aceton : Cumol = 1 : 1 : 0,38 einhält.

Die Zufuhr der zirkulierenden Produkte in einer Menge von 335 m $^3$ / h wird so eingestellt, daß die Größe des Verhältnisses $\Delta T_2 / \Delta T_1 = 21,4$ beträgt.

Die RMS wird in den Verdampfer gegeben, in dem das Gemisch aus Aceton, Cumol, Wasser und Phenol in einer Menge von 4400 kg / h unter einem Vakuum von 440 hPa (330 Torr) abgetrennt wird.

Dem Strom der aus dem unteren Teil des Verdampfers 3 austretenden Produkte werden 1700 kg / h Wasser zugeführt. Als Ergebnis der durchgeführten Veränderungen wird das Reaktionsmedium durch ein Molverhältnis von Phenol: Aceton : Cumol = 1 : 0,78 : 0,35 charakterisiert. Die Wasserkonzentration in den Produkten, die in den Spaltungsreaktor von DMPC und DCP kommen, beträgt 4,5 Gew.-%.

Der Prozeß der Spaltung von DMPC und DCP verläuft bei einer Temperatur von 168°C.

Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 3,19 | 0,21 | 0,116 | 0,71 | 0,31 | 0,33 | 950 | 70 |

Die Ausbeute an AMS beträgt 83,0 % d. Th. nach der Stufe der Spaltung von DCP und DMPC, die Bildung von phenolischem Teer 35,6 kg / t Phenol.

Den erhaltenen Spaltungsprodukten wird eine solche Menge Cumolfraktion zugeführt, daß der Gesamtgehalt an Cumol 40 Gew.-% beträgt. Die Cumolfraktion, die in einer Menge von 14200 kg / h als Kopfprodukt der Kolonne 7 abgetrennt wird, wird in die Stufe der Neutralisation geführt.

Die Neutralisation der Säure und die Abtrennung der Salze von den Produkten der Reaktion wird analog Beispiel 2 durchgeführt. Der Gehalt an Salzen in der RMS beträgt 18 ppm. Der Dampfaufwand in der Stufe der Abtrennung von Aceton und dem Gemisch aus Cumol-AMS beträgt 2,55 t / t Phenol.

Der Verbrauchskoeffizient Cumol / Phenol beträgt 1309 kg / t in der Stufe der Spaltung.

Als Ergebnis erhält man im Prozeß:

| Wertprodukte in kg / h | Phenol 25487,8 | | Aceton 15773,5 | α-Methylstyrol 1719,1 |
|---|---|---|---|---|
| Nebenprodukte am Austritt aus der Vorrichtung zur Spaltung von Cumolhydroperoxid in kg / t Phenol | | | | "phenolischer Teer" 35,6 |
| Dampfaufwand in t / t Phenol | bei der Destillation 1,03 | | bei der Spaltung 0,08 | bei der Abtrennung von Aceton und einem AMS-Cumol-Gemisch 2,55 |
| Gesamtaufwandskoeffizient Cumol / Phenol einschließlich der Crackung des "phenolischen Teers" in kg / t Phenol | | | | 1308 |

## Beispiel 9:

Der Prozeß wird analog zu Beispiel 5 durchgeführt, wobei das technische CHP die folgende Zusammensetzung hat:

| Zusammensetzung des technischen CHP | Benennung der Stoffe | | | | | Insgesamt |
|---|---|---|---|---|---|---|
| | Cumol | CHP | DMPC | ACP | DCP | |
| Gew.-% | 28,00 | 67,47 | 3,69 | 0,62 | 0,22 | 100 |
| t / h | 17,1679 | 41,3686 | 2,2625 | 0,3801 | 0,1349 | 61,314 |
| Gesamtselektivität, mol-% | | | | | | 93,1 |

Das in einer Menge von 61,314 t / h erhaltene technische CHP wird in die Stufe der Spaltung geführt, die in einem Reaktionsmedium durchgeführt wird, in dem ein Molverhältnis von Phenol-Aceton-Cumol = 1 : 1 : 0,55 eingehalten wird.

Die Zufuhr der zirkulierenden Produkte wird laut Formel (1)

$G_{zirk}$ = (480 x 61,314) / 28 = 1 051 t / h so eingestellt, daß der Umsatz CHP bei der Größe des Verhältnisses $\Delta T_2$ / $\Delta T_1$ = 3,16 pro Durchgang 76 % beträgt.

Die Zusammensetzung des Reaktionsmediums in dem Spaltungsreaktor von DCP und DMPC wird durch ein Molverhältnis von Phenol : Aceton : Cumol = 1 : 1,8 :

0,51 charakterisiert. Die Wasserkonzentration in den Produkten, die in den Spaltungsreaktor von DMPC und DCP kommen, beträgt 1,3 Gew.-%.

Der Prozeß der Spaltung von DMPC und DCP verläuft bei einer Temperatur von 150 °C.

Die Konzentration der wichtigsten Beimischungen und Nebenprodukte im Strom nach dem Spaltungsreaktor von DMPC und DCP ist unten dargestellt:

| Konzentration Nebenprodukte, Gew.-% | | | | | | Konzentration Beimischungen, ppm | |
|---|---|---|---|---|---|---|---|
| AMS | AMS-Dimer | DMPC | ACP | Cumyl-phenol | DCP | HA | MO |
| 2,55 | 0,24 | 0,02 | 0,55 | 0,30 | 0,01 | 870 | 55 |

Die Ausbeute an AMS beträgt 85,7 % d. Th. nach der Stufe der Spaltung von DCP und DMPC.

Den erhaltenen Spaltungsprodukten wird eine solche Menge Cumolfraktion zugeführt, daß der Gesamtgehalt an Cumol und AMS 40 Gew.-% beträgt. Die Cumolfraktion, die in einer Menge von 9434 kg / h als Kopfprodukt der Kolonne 7 abgetrennt wird, wird nur in die Stufe der Neutralisation geführt.

Die Neutralisation der Säure und die Abtrennung der Salze von den Produkten der Reaktion wird analog Beispiel 2 durchgeführt. Der Gehalt an Salzen in der RMS beträgt 19 ppm.

Der Dampfaufwand in der Stufe der Abtrennung von Aceton und dem Gemisch aus Cumol-AMS beträgt 2,46 t / t Phenol.

Der Verbrauchskoeffizient Cumol / Phenol beträgt 1306 kg / t in der Stufe der Spaltung.

Als Ergebnis erhält man im Prozeß:

| Wertprodukte in kg / h | Phenol 25634,4 | Aceton 12518,5 | α-Methylstyrol 1752,6 |
|---|---|---|---|
| Nebenprodukte am Austritt aus der Vorrichtung zur Spaltung von Cumolhydroperoxid in kg / t Phenol | | | "phenolischer Teer" 30,3 |
| Dampfaufwand in t / t Phenol | bei der Destillation 0,94 | bei der Spaltung 0,08 | bei der Abtrennung von Aceton und dem AMS-Cumol-Gemisch 2,46 |
| Gesamtaufwandskoeffizient Cumol / Phenol einschließlich der Crackung des "phenolischen Teers" in kg / t Phenol | | | 1305 |

## Tabelle 3: Zusammenfassung der Beispiele

| NN | Aufwand, t/h | | Verlust der Selektivitaet beim Auf-konzentrie-ren von CHP | Zusammensetzung des Reaktions-mediums der Spaltung als Molver-haeltnis Phenol:Aceton:Cumol | |
|---|---|---|---|---|---|
| | techn CHP | Gzirk. | % CHP | Spaltung von CHP | Spaltung von DCP und DMPC |
| 1 | 2 | 3 | 4 | 5 | 6 |
| 1 | 50.5 | | 0.47 | 1 : 1.36 : 0.2 | 1 : 1.36 : 0.2 |
| 2 | 63 | 1008 | 0 | 1 : 1 : 0.61 | 1 : 0.78 : 0.87 |
| 3 | 61.314 | 1051 | 0.07 | 1 : 1 : 0.55 | 1 : 0.78 : 0.70 |
| 4 | 55.9 | 1278 | 0.17 | 1 : 1 : 0.38 | 1 : 0.77 : 0.61 |
| 5 | 61.34 | 1051 | 0.07 | 1 : 1 : 0.55 | 1 : 1 : 0.53 |
| 6 | 58.09 | 1328 | 0.17 | 1 : 1 : 0.42 | 1 : 1 : 0.39 |
| 7 | 55.9 | 1278 | 0.17 | 1 : 1 : 0.38 | 1 : 0.77 : 0.61 |
| 8 | 55.9 | 335 | 0.17 | 1 : 1 : 0.38 | 1 : 0.77 : 0.35 |
| 9 | 55.94 | 1051 | 0.07 | 1 : 1 : 0.55 | 1 : 0.80 : 0.51 |

## Tabelle 3 (Fortsetzung)

| NN | Tempe-ratur der Spaltung von DCP oC | $dT_2/dT_1$ | Cumolgehalt. Gew.-% | | | | Zusatz von Cumol und Wasser bei der Spaltungvon DCP und DMPC kg / t CHP | |
|---|---|---|---|---|---|---|---|---|
| | | | techn. CHP | Spaltung von CHP | Spal-tung von DCP | Neutra-lisation | Cumol | Wasser |
| 1 | 7 | 8 | 9 | 10 | 11 | 12 | 13 | 14 |
| 1 | 100 | 7.6 | 12.3 | 11.46 | 11.39 | 11 | 0 | 7.1 |
| 2 | 168 | 2.3 | 30 | 30.00 | 39.89 | 39.89 | 159 | 15.8 |
| 3 | 160 | 1.5 | 28 | 28.00 | 35 | 37.52 | 101 | 14.67 |
| 4 | 151 | 3.8 | 21 | 21.00 | 32 | 37.38 | 152 | 17.8 |
| 5 | 156 | 2.3 | 28 | 28.00 | 27.4 | 37.66 | 0 | 23.7 |
| 6 | 168 | 5.9 | 21 | 21.00 | 20.58 | 35.12 | 0 | 19.9 |
| 7 | 151 | 5.7 | 21 | 21.00 | 32 | 37.45 | 152 | 10.3 |
| 8 | 168 | 21.4 | 21 | 21.00 | 21.15 | 37.63 | 0 | 30.4 |
| 9 | 150 | 3.16 | 28 | 28.00 | 29 | 37.49 | 0 | 1.03 |

Tabelle 3 (Fortsetzung)

| NN | Umsatz von DCP % | Aus- beute von AMS % theor | Aus beute von phenol. Teer kg/t Phenol | Aufwands- koeffizient bei der Spaltung, kg/t Phenol | Konzen- tration an Beimi- schungen, ppm | |
|---|---|---|---|---|---|---|
| | | | | | MO | HA |
| 1 | 15 | 16 | 17 | 18 | 19 | 20 |
| 1 | 92.5 | 75.9 | 44 | 1318.4 | 300 | 1500 |
| 2 | 99.87 | 90.6 | 24.4 | 1300 | 70 | 950 |
| 3 | 98.41 | 90.28 | 25.4 | 1301 | 60 | 920 |
| 4 | 99.46 | 88.59 | 28.1 | 1303 | 50 | 890 |
| 5 | 99.98 | 88.31 | 27.7 | 1302 | 55 | 910 |
| 6 | 99.99 | 85.47 | 60.4 | 1334 | 70 | 950 |
| 7 | 99.82 | 87.6 | 28.8 | 1305 | 50 | 890 |
| 8 | 91.48 | 84 | 36 | 1309 | 70 | 950 |
| 9 | 99.71 | 85.74 | 30.3 | 1306 | 50 | 870 |

Tabelle 3 (Fortsetzung)

| NN | Dampfaufwand, t/t Phenol | | | Zusätzliche Stoff- zufuhr aus der Neutralisations- stufe, kg / t CHP | | Stoffgehalt nach der Neutralisationsstufe | |
|---|---|---|---|---|---|---|---|
| | Aufkonzen- trieren von CHP | Rektifi- kation | Gesamt- aufwand | Wasser | Cumol | Wasser Gew.-% | Na2SO4, ppm |
| 1 | 21 | 22 | 23 | 24 | 25 | 26 | 27 |
| 1 | 1.12 | 2.9 | 4.02 | 112 | 0 | 12 | 790 |
| 2 | 0.93 | 2.46 | 3.39 | 11 | 0 | 3.5 | 3 |
| 3 | 0.94 | 2.46 | 3.4 | 9.6 | 42.4 | 3.5 | 20 |
| 4 | 1.03 | 2.55 | 3.58 | 12 | 102 | 3.5 | 15 |
| 5 | 0.94 | 2.46 | 3.4 | 18 | 178 | 3.5 | 18 |
| 6 | 1.03 | 2.8 | 3.83 | 14 | 236 | 3.5 | 8 |
| 7 | 1.03 | 2.55 | 3.58 | 19 | 100 | 3.5 | 16 |
| 8 | 1.03 | 2.55 | 3.58 | 0 | 254 | 3.5 | 18 |
| 9 | 0.94 | 2.46 | 3.49 | 23.81 | 149 | 3.5 | 19 |

**Bezugszeichenliste**

| | |
|---|---|
| 1 | Destillationsstufe (1 A, B) |
| 2 | CHP-Spaltungsreaktor (2 A, B, C) |
| 3 | Verdampfer |
| 4 | DCP / DMCP-Spaltungsreaktor |
| 5 | Neutralisator |
| 6 | Aceton-Abtrennungskolonne |

| 7 | Cumol-Abtrennungskolonne |
| 8 | Phenol / Cumol / AMS-Trennkolonne |
| 9 | Rückleitung für Cumol zur Oxidation (Strom II) |
| 10 | Zuleitung von Cumol-Oxidationsprodukten (Strom I) |
| 11 | Leitung für die konzentrierten Cumol-Oxidationsprodukte |
| 12 | Kühlwasserleitung |
| 13 | Mischer für Oxidationsprodukte, Rücklauf und Katalysator |
| 14 | Pumpe |
| 15 | Katalysator($H_2SO_4$)-Zuleitung |
| 16 | Kalorimeter $\Delta T_1$ (Minirohrreaktor) |
| 17 | Acetonableitung |
| 18 | Cumol / AMS-Rückleitung zur Hydrierung |
| 19 | Neutralisationsmittel(NaOH)-Zuleitung |
| 20 | Neutralisationszuleitung |
| 21 | Neutralisationsableitung (Strom VI) |
| 22 | Abwasserableitung |
| 23 | Neutralisationsumlaufpumpe |
| 24 | Rohacetonableitung (Strom VII) |
| 25 | Rohphenolleitung (Strom VIII) |
| 26 | Cumol-Rückführungsleitungen (Strom IV, IVa, IVb) |
| 27, 27' | Separator für Cumol / $H_2O$ |
| 28 | Rückleitung für Wasser (Strom III) |
| 29 | Phenol / Cumol / AMS-Leitung |
| 30 | Phenol / Teer-Ableitung zur Rektifikation |

**Patentansprüche**

1. Verbessertes Verfahren zur Herstellung von Phenol und Aceton durch Oxidation von Cumol zu technischem Cumolhydroperoxid (CHP) unter katalytischer Spaltung des CHP, **dadurch gekennzeichnet**, daß die Oxidationsprodukte bis zu einem Cumolgehalt im technischen Cumolhydroperoxid von 21 bis zu 30 Gew.-% konzentriert werden und dieses Gemisch in die katalytische Spaltung eingesetzt wird und bei der Spaltung ein Molverhältnis von Phenol: Aceton:Cumol von 1:1-0,77:0,35-0,87 eingehalten wird.

2. Verfahren nach Anspruch 1 **dadurch gekennzeichnet**, daß man die Spaltung des technischen Cumolhydroperoxids in Mischreaktoren und die Spaltung des als Nebenprodukt gebildeten Dicumylperoxids in einem Rohrreaktor bei unterschiedlichen Zusammensetzungen des Reaktionsmediums in oben erwähnten Reaktoren durchführt.

3. Verfahren nach Anspruch 2, **dadurch gekennzeichnet**, daß im Reaktionsmedium in der Stufe der Spaltung von technischem Cumolhydroperoxid im Mischreaktor ein Molverhältnis von Phenol : Aceton : Cumol 1 : 1 : (0,38 - 0,61) eingehalten wird.

4. Verfahren nach Anspruch 2, **dadurch gekennzeichnet**, daß für die Spaltung des technischen Cumolhydroperoxids im Mischreaktor je nach Gehalt des enthaltenen Cumols das folgende Verhältnis eingehalten wird:

$$G_{zirk} = \frac{480 \times G_{tCHP}}{\% \text{ Cumol}} \tag{1}$$

wobei $G_{zirk}$ die Menge der zirkulierenden Spaltungsprodukte (t / h), $G_{tCHP}$ die Menge des technischen CHP zugeführt in die Spaltung (t / h) und % Cumol Gew.-% Cumol im technischen CHP darstellt.

5. Verfahren nach Anspruch 2, **dadurch gekennzeichnet**, daß die Spaltung von Dicumylperoxid und Dimethylphenylcarbinol im Rohrreaktor in einem Reaktionsmedium mit einem Molverhältnis Phenol : Aceton : Cumol = 1 : (1 - 0,77): (0,35 - 0,87) vorzugsweise bei Temperaturen von 150 - 168°C durchgeführt wird.

6. Verfahren nach Anspruch 5, **dadurch gekennzeichnet**, daß in den Spaltungsreaktor von Dicumylperoxid und Dimethylphenylcarbinol die Cumolfraktion in einer Menge bis zu 160 kg / t von technischem Cumolhydroperoxid zugeführt wird.

**7.** Verfahren nach Ansprüchen 5 und 6, **dadurch gekennzeichnet**, daß im Spaltungsreaktor von Dicumylperoxid und Dimethylphenylcarbinol das Wasser in einer Menge von 1 - 30,4 kg / t von technischem Cumolhydroperoxid zugeführt wird.

**8.** Verfahren nach Ansprüchen 2 - 7, **dadurch gekennzeichnet**, daß die Steuerung des Prozesses der Spaltung von Dicumylperoxid und Dimethylphenylcarbinol durch Regulieren der Größe des Verhältnisses ($\Delta T_2 / \Delta T_1$) im Bereich von 1,5 bis 21,4 - vorzugsweise 3 - 8 - in der Vorrichtung geschieht, wobei:

$\Delta T_2$ die Temperaturdifferenz der Produkte am Eintritt und Austritt des Spaltungsreaktors von Dicumylperoxid und Dimethylphenylcarbinol, und

$\Delta T_1$ die Temperaturdifferenz der Produkte am Eintritt und Austritt des Kalorimeters, das in der Leitung der Zirkulation von Spaltungsprodukten des technischen Cumolhydroperoxid installiert wird, ist.

**9.** Verfahren nach Anspruch 5, **dadurch gekennzeichnet,** daß die Spaltung, von Dicumylperoxid und Dimethylphenylcarbinol im Rohrreaktor in einem Reaktionsmedium mit gesenktem Gehalt an Aceton durchgeführt wird, wobei der gesenkte Gehalt von Aceton entweder durch Entfernung eines Teils des Acetons, oder mit Hilfe der Zufuhr einer zusätzlichen Menge Cumol- und Wasserfraktion in die Spaltungsprodukte, oder durch Verwendung beider Verfahren erreicht wird.

**10.** Verfahren nach Anspruch 9, **dadurch gekennzeichnet**, daß in der Stufe der Neutralisation der Schwefelsäure der Gesamtgehalt an Cumol und α—Methylstyrol in den Spaltungsprodukten durch die Zufuhr der Kohlenwasserstoffraktion 40 Gew.-%, der Wassergehalt 3,5 Gew.-%, und die Konzentration der Salze in den Spaltungsprodukten nach der Stufe der Neutralisation der Schwefelsäure 3 bis 20 ppm beträgt.

**Claims**

**1.** Improved process for the production of phenol and acetone by oxidation of cumene to technical cumene hydroperoxide (CHP) with catalytic cleavage of the CHP, characterised in that the oxidation products are concentrated up to a cumene content in the technical cumene hydroperoxide of 21 to 30 wt.% and this mixture is used in the catalytic cleavage and, in the case of the cleavage, a mole ratio of phenol:acetone:cumene of 1:1 - 0.77:0.35 - 0.87 is maintained.

**2.** Process according to claim 1, characterised in that one carries out the cleavage of the technical cumene hydroperoxide in mix reactors and the cleavage of the dicumyl peroxide formed as by-product in a tubular reactor with differing compositions of the reaction medium in the above-mentioned reactors.

**3.** Process according to claim 2, characterised in that, in the reaction medium in the step of the cleavage of technical cumene hydroperoxide, in the mix reactor is maintained a mole ratio of phenol:acetone:cumene of 1:1:(0.38 - 0.61).

**4.** Process according to claim 2, characterised in that, for the cleavage of the technical cumene hydroperoxide, in the mix reactor is maintained, depending upon the content of the cumene obtained, the following ratio:

$$G_{circ} \frac{480 \times G_{tCHP}}{\% \text{ cumene}} \quad (1)$$

whereby $G_{circ}$ represents the amount of the circulating cleavage products (t/h), $G_{tCHP}$ the amount of the technical CHP supplied to the cleavage (t/h) and % cumene wt.% in the technical CHP.

**5.** Process according to claim 2, characterised in that the cleavage of dicumyl peroxide and dimethylphenylcarbinol is carried out in the tubular reactor in a reaction medium with a mole ratio of phenol: acetone:cumene = 1:(1 - 0.77):(0.35 - 0.87), preferably at temperatures of 150 - 168°C.

**6.** Process according to claim 5, characterised in that, into the cleavage reactor of dicumyl peroxide and dimethylphenylcarbinol, the cumene fraction is supplied in an amount of up to 160 kg/t of technical cumene hydroperoxide.

7. Process according to claims 5 and 6, characterised in that, into the cleavage reactor of dicumyl peroxide and dimethylphenylcarbinol, the water is supplied in an amount of 1 - 30.4 kg/t of technical cumene hydroperoxide.

8. Process according to claims 2 - 7 , characterised in that the control of the process of the cleavage of dicumyl peroxide and dimethylphenylcarbinol takes place by regulation of the value of the ratio ($\Delta T_2/\Delta T_1$) in the range of 1.5 to 21.4, preferably 3 - 8, in the device, whereby:

$\Delta T_2$ is the temperature difference of the product at the inlet and outlet of the cleavage reactor of dicumyl peroxide and dimethylphenylcarbinol, and
$\Delta T_1$ is the temperature difference of the products at the inlet and outlet of the calorimeter which is installed in the pipe of the circulation of cleavage products of the technical cumene hydroperoxide.

9. Process according to claim 5, characterised in that the cleavage of dicumyl peroxide and dimethylphenylcarbinol is carried out in the tubular reactor in a reaction medium with lowered content of acetone, whereby the lowered content of acetone is achieved either by removal of a part of the acetone or with the help of the introduction of an additional amount of cumene and water fraction into the cleavage products or by use of both processes.

10. Process according to claim 9, characterised in that, in the step of the neutralisation of the sulphuric acid, the total content of cumene and $\alpha$-methylstyrene in the cleavage products, by the supply of the hydrocarbon fraction, amounts to 40 wt.%, the water content to 3.6 wt.% and the concentration of the salts in the cleavage products after the step of the neutralisation of the sulphuric acid to 3 to 20 ppm.

## Revendications

1. Procédé amélioré pour la préparation de phénol et d'acétone par oxydation de cumène en hydroperoxyde de cumène (HPC) de qualité technique sous dissociation catalytique de l'HPC, caractérisé en ce que les produits d'oxydation sont concentrés jusqu'à une teneur en cumène dans l'hydroperoxyde de cumène de qualité technique de 21 à 30 % en poids et en ce qu'on utilise ce mélange dans la dissociation catalytique et en ce qu'on maintient un rapport molaire phénol : acétone : cumène de 1 : 1-0,77 : 0,35-0,87 lors de la dissociation.

2. Procédé selon la revendication 1, caractérisé en ce qu'on effectue la dissociation de l'hydroperoxyde de cumène de qualité technique dans des réacteurs mélangeurs et la dissociation du peroxyde de dicumyle formé comme produit secondaire dans un réacteur tubulaire avec des compositions différentes du milieu réactionnel dans les réacteurs susmentionnés.

3. Procédé selon la revendication 2, caractérisé en ce qu'on maintient dans le milieu réactionnel dans l'étape de la dissociation de l'hydroperoxyde de cumène de qualité technique dans le réacteur mélangeur un rapport molaire phénol : acétone : cumène de 1 : 1 : (0,38-0,61).

4. Procédé selon la revendication 2, caractérisé en ce qu'on maintient, pour la dissociation de l'hydroperoxyde de cumène de qualité technique dans le réacteur mélangeur, en fonction de la teneur en cumène contenu, le rapport suivant :

$$G_{circ} = \frac{480 \ x \ G_{tHPC}}{\% \ cumène} \qquad (1)$$

$G_{circ}$ représentant la quantité de produits de dissociation en circulation (T/h), $G_{tHPC}$ la quantité de l'HPC de qualité technique alimentée dans la dissociation (T/h) et % cumène le pourcentage en poids de cumène dans l'HPC de qualité technique.

5. Procédé selon la revendication 2, caractérisé en ce que la dissociation du peroxyde de dicumyle et du diméthylphénylcarbinol dans le réacteur tubulaire est effectuée dans un milieu réactionnel présentant un rapport molaire phénol : acétone : cumène = 1 : (1-0,77) : (0,35-0,87), de préférence à des températures de 150-168 °C.

**6.** Procédé selon la revendication 5, caractérisé en ce qu'on alimente la fraction de cumène en une quantité jusqu'à 160 kg/T d'hydroperoxyde de cumène de qualité technique dans le réacteur de dissociation du peroxyde de dicumyle et du diméthylphénylcarbinol.

**7.** Procédé selon les revendications 5 et 6, caractérisé en ce qu'on alimente l'eau en une quantité de 1 - 30,4 kg/T d'hydroperoxyde de cumène de qualité technique dans le réacteur de dissociation du peroxyde de dicumyle et de diméthylphénylcarbinol.

**8.** Procédé selon l'une quelconque des revendications 2 à 7, caractérisé en ce que la commande du processus de la dissociation du peroxyde de dicumyle et de diméthylphénylcarbinol est effectuée par la régulation de la grandeur du rapport ($\Delta T_2/\Delta T_1$) dans la plage de 1,5 à 21,4, de préférence de 3 - 8, dans le dispositif,

$\Delta T_2$ représentant la différence de température des produits à l'entrée et à la sortie du réacteur de dissociation du peroxyde de dicumyle et du diméthylphénylcarbinol et

$\Delta T_1$ représentant la différence de température des produits à l'entrée et à la sortie du calorimètre, qui est installé dans la conduite de circulation des produits de dissociation de l'hydroperoxyde de cumène de qualité technique.

**9.** Procédé selon la revendication 5, caractérisé en ce que la dissociation du peroxyde de dicumyle et du diméthylphénylcarbinol dans le réacteur tubulaire est réalisée dans un milieu réactionnel avec une teneur réduite en acétone, la teneur réduite en acétone étant obtenue soit par élimination d'une partie de l'acétone, ou à l'aide de l'alimentation d'une quantité supplémentaire de fraction de cumène et d'eau dans les produits de dissociation, ou par la mise en oeuvre des deux procédés.

**10.** Procédé selon la revendication 9, caractérisé en ce que, dans l'étape de neutralisation de l'acide sulfurique, la teneur totale en cumène et en αméthylstyrène dans les produits de dissociation, par l'alimentation de la fraction hydrocarbonée, est de 40 % en poids, la teneur en eau de 3,5 % en poids et la concentration des sels dans les produits de dissociation, après l'étape de neutralisation de l'acide sulfurique, de 3 à 20 ppm.

Fig. 1: Abhängigkeit der Reaktionskonstante K2 der Spaltung von CHP bei wechselnder Konzentration von Cumol in Phenol-Aceton-Medium.

Fig. 2: Abhängigkeit der Reaktionskonstante K2 der Spaltung von CHP bei wechselndem Verhältnis von Phenol zu Aceton

Fig. 3: Abhängigkeit der Reaktionskonstante K2 der Spaltung von CHP bei wechselnder Wasserkonzentration

Ausbeute von AMS

Bildung von phenolischem
Teer. kg t Phenol

Fig. 4: Abhängigkeit der Ausbeute von AMS
und phenolischem Teer, bezogen auf 1 t
Phenol, bei unterschiedlichem DCP-Umsatz

T=50°C

| [Cumol] Gew.-% | [H2O] Gew.-% | [Na2SO4] Gew.-% |
|---|---|---|
| 0 | 15.8 | 0.1950 |
| 5 | 12.0 | 0.1170 |
| 10 | 10.3 | 0.0773 |
| 20 | 7.5 | 0.0250 |
| 30 | 5.0 | 0.0080 |
| 40 | 3.5 | 0.0002 |

Konzentration von Cumol, Gew.-%

Konzentration von Aceton. Gew.-%

Fig. 5: Abhängigkeit des Gehalts an gelöstem
Na2SO4 in der Reaktionsmasse der Spaltung
von dem darin enthaltenen Cumol

Fig. 6: Abhängigkeit des Gehalts an gelöstem
Wasser in der Reaktionsmasse der Spaltung
von dem darin enthaltenen Cumol

Fig. 7

EP 0 944 567 B1